# EUROPEAN PATENT SPECIFICATION

(11) **EP 4 267 144 B1**
(45) Date of publication and mention of the grant of the patent: **31.12.2025**
(21) Application number: 21909623.7
(22) Date of filing: 19.11.2021
(51) Int. Cl.: A61K 31/52, A61K 31/519, A61K 31/47, A61K 31/472, A61P 17/02, A61K 31/44, A61P 17/10

(54) **TOPICAL BRAF INHIBITOR COMPOSITIONS FOR TREATMENT OF EGFR DOWNSTREAM EFFECTORS - INDUCED REACTIONS**
TOPISCHE BRAF-HEMMER-ZUSAMMENSETZUNGEN ZUR BEHANDLUNG VON EGFR-ABWÄRTSEFFEKTOREN - INDUZIERTEN REAKTIONEN
COMPOSITIONS D'INHIBITEUR DE BRAF TOPIQUES POUR LE TRAITEMENT DE RÉACTIONS INDUITES PAR DES EFFECTEURS EN AVAL D'EGFR

(30) Priority: 24.12.2020 US 202063130483 P
(43) Date of publication of application: 01.11.2023
(73) Proprietor: Lutris Pharma Ltd., 6971039 Tel Aviv (IL)
(72) Inventor: SHELACH, Noa, 6971039 Tel Aviv (IL); ZELINGER, Galit, 6971039 Tel Aviv (IL)
(74) Representative: Dennemeyer & Associates S.A.
(86) International application number: PCT/IB2021/000799
(87) International publication number: WO 2022/136912

(56) References cited:
- WO-A1-2019/070920
- WO-A1-2020/165755
- WO-A2-2011/026076
- WO-A2-2019/026065

## Description

### RELATED APPLICATIONS

This application claims priority to and benefit of U.S. Provisional Patent Application Serial No. 63/130,483, filed on December 24, 2020.

### FIELD OF THE INVENTION

The present disclosure relates to a compound of Formula IV, i.e. LUT014, for treating EGFR downstream effectors-induced cutaneous reactions and, in particular, Epidermal Growth Factor Receptor (EGFR) inhibitor-induced cutaneous reactions.

### BACKGROUND

Abnormal activation of Epidermal Growth Factor Receptor (EGFR) pathway is involved in various diseases, in particular, in several types of cancers such as lung cancer, colorectal cancer, head and neck cancer and pancreatic cancer. EGFR antagonists such as monoclonal antibodies (e.g., cetuximab, panitumumab) and small molecule tyrosine kinase inhibitors (e.g. gefitinib, erlotinib, lapatinib) are used for treating many EGFR-mediated cancers. While these EGFR antagonists are useful for treating cancer, they are also associated with severe cutaneous side effects.

WO2019/026065 discloses a topical composition containing 1% to 5% w/w of LUT014 for treating or preventing cutaneous adverse reactions, including acneiform rash, caused by EGFR, PI3K, or MEK inhibitors.

### SUMMARY OF THE INVENTION

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy.

The present invention relates to a topical pharmaceutical composition for use in a method of treating and/or preventing EGFR inhibitor, PI3K inhibitor, MEK inhibitor and/or HER dimerization inhibitor - induced acneiform lesions in a subject in need thereof, the method comprising:administering the topical pharmaceutical composition once a day for 4 to 6 weeks to the skin of the subject in need thereof, the topical pharmaceutical composition comprising from 0.03 % weight/weight to 0.1 % weight/weight of a compound of Formula IV and a pharmaceutically acceptable carrier or excipient.

In some embodiments, the topical pharmaceutical composition is in the form of a gel, a hydrogel, an ointment, a cream, a spray, a dermal patch, a foam, a lotion or a liquid. In some embodiments, the topical pharmaceutical composition is in the form of a gel. In some embodiments, the topical pharmaceutical composition is in the form of a spray.

In some embodiments, the acneiform lesions are EGFR inhibitor induced acneiform lesions. In some embodiments, the subject in need thereof is a subject has a cancer treated with EGFR inhibitor, PI3K inhibitor, MEK inhibitor and/or HER dimerization inhibitor.

In some embodiments, the subject in need thereof has grade 1 acneiform lesions. In some embodiments, the subject in need thereof has grade 2 acneiform lesions. In some embodiments, the subject in need thereof has grade 3 acneiform lesions. In some embodiments, the subject in need thereof has grade 4 acneiform lesions.

In some embodiments, the method reduces the severity or prevents escalation of the acneiform lesions. In some embodiments, the method reduces the severity of the acneiform lesions from grade 4 to grade 3, 2, 1, or 0. In some embodiments, the method reduces the severity of the acneiform lesions from grade 3 to grade 2, 1, or 0. In some embodiments, the method reduces the severity of the acneiform lesions from grade 2 to grade 1, or 0. In some embodiments, the method reduces the severity of the acneiform lesions from grade 1 to grade 0.

In some embodiments, the acneiform lesions are induced by treatment with gefitinib, erlotinib, lapatinib, cetuximab, panitumumab, vandetanib, necitumumab, osimertinib, Afatinib, Neratinib, Dacomitinib, Pertuzumab or combinations thereof.

Aspects of the disclosure relate to a topical pharmaceutical composition comprising from 0.03 % w/w to 0.1 % w/w of a compound of Formula IV 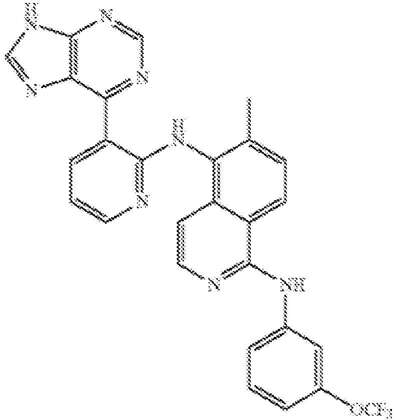 and a pharmaceutically acceptable carrier or excipient.

In some embodiments, the composition is in the form of a gel, a hydrogel, an ointment, a cream, a foam, a spray, a lotion, a liquid or a dermal patch. In some embodiments, the topical composition comprises about 0.03 % weight/weight of the compound of Formula IV.

Aspects of the disclosure relate to a topical pharmaceutical composition comprising from 0.03% w/w to 0.1 % w/w of a compound of Formula IV and a pharmaceutically acceptable carrier or excipient for use in a method of treating and/or preventing EGFR inhibitor, PI3K inhibitor, MEK inhibitor and/or HER dimerization inhibitor -induced acneiform lesions in a subject in need thereof.

Aspects of the disclosure relate to a topical pharmaceutical composition comprising from 0.03 % w/w to 0.1 % w/w of a compound of Formula IV and a pharmaceutically acceptable carrier or excipient for use in a method of treating and/or preventing EGFR inhibitor, PI3K inhibitor, MEK inhibitor and/or HER dimerization inhibitor -induced acneiform lesions in a subject in need thereof, wherein the topical pharmaceutical composition is administered topically for four to six weeks to the skin of the subject in need thereof.

### BRIEF DESCRIPTION OF THE DRAWINGS

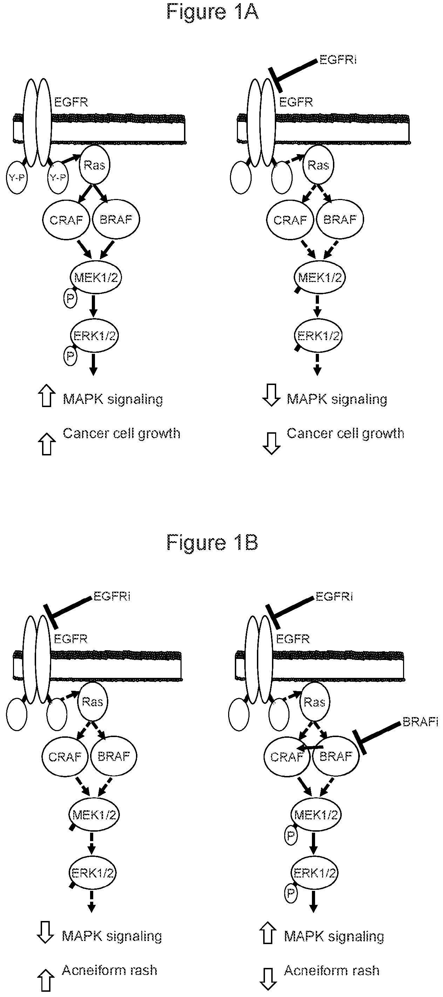

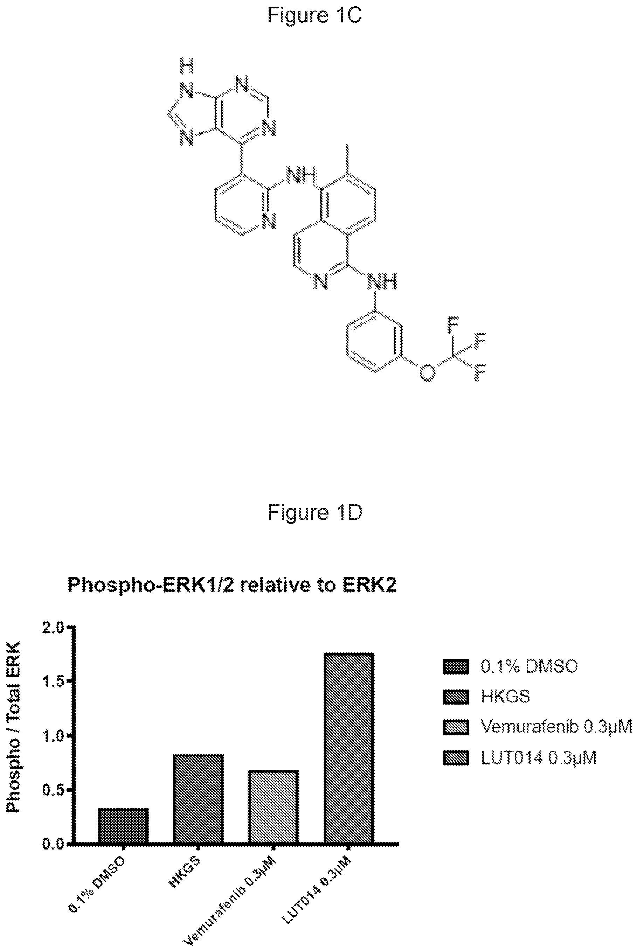

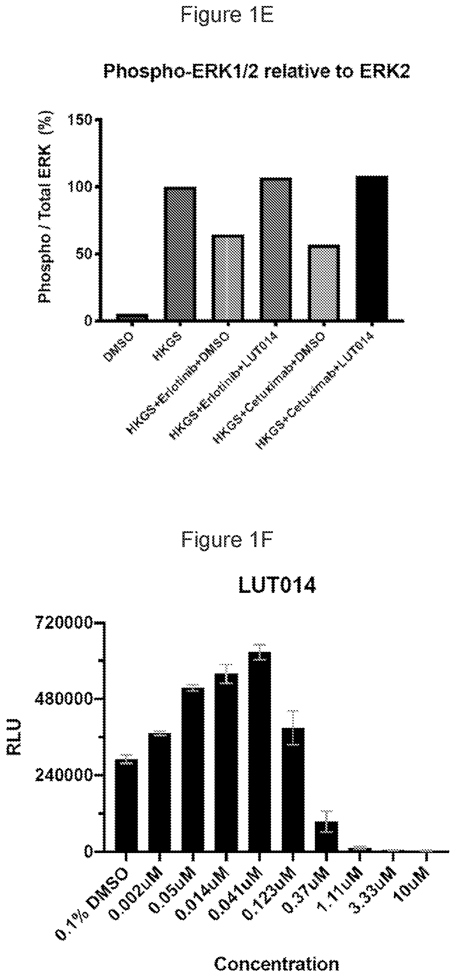

**FIGs. 1A-1F** show the paradoxical MAPK activation, reversion of EGFR inhibition and cellular proliferation with LUT014. FIG. 1A and FIG. 1B depict hypothesis of the mechanism of action of topical LUT014 to reverse the pathogenesis of acneiform rash induced by EGFR inhibitor therapy. In cancer cells (FIG. 1A), oncogenic EGFR signaling leads to increased MAPK signaling and cancer cell proliferation. With EGFR inhibitor treatment, the MAPK signaling pathway is inhibited resulting in tumor shrinkage. In skin cells (FIG. 1B), administration of EGFR inhibitor treatment leads to decreased MAPK signaling and acneiform rash. Topical therapy with LUT014 would override the MAPK pathway inhibition, resulting in an increase in pERK signaling in skin cells. FIG. 1C shows the chemical structure of LUT014. FIG. 1D shows the effects of LUT014 on MAPK signaling in primary adult human epidermal keratinocytes (HEKa) cells. Western blot analysis of HEKa lysates for phosphorylated ERK1/2 and total ERK2 following 2-hour exposure to DMSO vehicle control, human keratinocyte growth supplement (HKGS) to stimulate the MAPK pathway, the positive control BRAF inhibitor vemurafenib and LUT014. The bar graph shows the quantified ratio of phosphorylated ERK1/2 relative to total ERK2. FIG. 1E shows the reversal of EGFR inhibitor-mediated pERK inhibition with LUT014. HEKa cells were cultured with HKGS to increase pERK, and exposed to the presence of the EGFR inhibitors erlotinib or cetuximab, without or with the addition of LUT014. Cell lysates were analyzed by Western blot for phosphorylated ERK1/2 and total ERK2. FIG. 1F shows the bell-shaped curve of paradoxical proliferation with increasing concentrations of LUT014. Proliferation assay of MIA-PaCa-2 cells treated with various concentrations of LUT014 for 72 hours.
**FIGs 2A-2D** show the efficacy of topical LUT014 for EGFR inhibitor-induced acneiform rash. FIG. 2A are pictures of baseline and on-therapy areas of rash in patient 104001 from cohort 1, after 1 week of treatment with LUT014. FIGS 2B-2D show the evolution of rash following the CTCAE scale (FIG. 2B), MASCC (FIG. 2C) and FACT-13 (FIG. 2D).
**FIG. 3** shows the CONSORT diagram of the clinical trial.
**FIG. 4A-4B** shows the pharmacokinetic analyses: Plasma levels of LUT014 measured using liquid chromatography tandem mass spectroscopy (LC-MS/MS) at day 0 (FIG. 4A) and day 7 (FIG. 4B).

### DETAILED DESCRIPTION

Various embodiments are described hereinafter.

Unless defined otherwise, all technical and scientific terms used herein have the same meaning as commonly understood to one of ordinary skill in the art to which this invention pertains. Otherwise, certain terms used herein have the meanings as set forth in the specification. It must be noted that as used herein and in the appended claims, the singular forms "a," "an," and "the" include plural references unless the context clearly dictates otherwise.

The term "about" when used before a numerical designation, e.g., temperature, time, amount, and concentration, including range, indicates approximations which may vary by (+) or (-) 10%, 5% or 1%.

The terms "pharmaceutically acceptable salts" or "salts thereof" mean salts which are pharmaceutically acceptable, and which possess the desired pharmacological activity.

The terms "treat", "treating" or "treatment", as used herein, include alleviating, abating or ameliorating a disease or condition or one or more symptoms thereof, preventing additional symptoms, ameliorating or preventing the underlying metabolic causes of symptoms, inhibiting the disease or condition, e.g., arresting or suppressing the development of the disease or condition, relieving the disease or condition, causing regression of the disease or condition, relieving a condition caused by the disease or condition, or suppressing the symptoms of the disease or condition, and are intended to include prophylaxis. The terms also include relieving the disease or conditions, e.g., causing the regression of clinical symptoms. The terms further include achieving a therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the individual, notwithstanding that the individual is still be afflicted with the underlying disorder. For prophylactic benefit, the compositions are administered to an individual at risk of developing a particular disease, or to an individual reporting one or more of the physiological symptoms of a disease, even though a diagnosis of this disease has not been made.

The term "carrier" as used herein, refers to nontoxic chemical compounds or agents that facilitate the incorporation of a compound into cells, e.g., dermal cells, or tissues. Carriers useful herein include any such materials known in the art, which are nontoxic and do not interact with other components of the formulation in which it is contained in a deleterious manner. As used herein, "pharmaceutically acceptable carrier" includes any and all solvents, dispersion media, and the like, which are pharmaceutically acceptable.

The term "therapeutically effective amount" of a compound or a composition refers to that amount of the compound or the composition that results in treatment, including reduction or inhibition of symptoms in a patient.

The term "subject" as used herein refers to organisms to be treated by the compounds or compositions of the present disclosure. Such organisms include a mammal, including a human or non-human mammal. The terms "patient", "individual" and "subject" may be used interchangeably.

Provided herein are methods of treating EGFR-induced cutaneous reactions using the topical compositions of the present disclosure. An adverse effect of downstream EGFR effectors like EGFR inhibitor, Phosphoinositide-3-kinase (PI3K) inhibitor, Mitogen-activated protein kinase kinase (MEK) inhibitor and/or human EGF receptor (HER) dimerization inhibitor is cutaneous reactions. Cutaneous adverse reactions to EGFR pathway inhibition include acneiform (papulopustular) rash, abnormal scalp, facial hair and/or eyelash growth, paronychia with or without pyogenic granulomas and telangiectasia.

Various kinases such as phosphatidylinositol-3-kinases (also referred herein as Phosphoinositide-3-kinases, PI3-kinases or PI3K), mitogen-activated protein kinases (MAPK), and kinases upstream of mitogen-activated protein kinase (MAPK) such as mitogen-activated protein kinase kinases MEK and MKK, act as downstream effectors of EGFR and many other receptor tyrosine kinases and are involved in cellular functions such as cell growth, proliferation, differentiation, motility, survival, and intracellular trafficking. Pertuzumab (Perjeta^{®}) is a monoclonal antibody which binds to the domain II of HER2, which is essential for dimerization. Pertuzumab is a HER dimerization inhibitor which inhibits dimerization of HER2 to HER3 and to EGFR. Therapeutic agents that target these pathways are also used in the treatment of a number of proliferative diseases, such as melanoma, lung cancer, colorectal cancer, brain cancer, multiple myeloma, breast cancer, pancreatic cancer and neurofibromatosis. Exemplary therapeutic agents that target these pathways include kinase inhibitors such as Trametinib (Mekinist^{®}) and Cobimetinib (Cotellic^{®}) mitogen-activated protein kinase (MEK) inhibitors. However, inhibitors of these kinases are also associated with adverse side effects. For example, cutaneous adverse events caused by MEK inhibitors have been reported, and include acneiform (papulopustular) rash, abnormal scalp, facial hair and/or eyelash growth, paronychia with or without pyogenic granulomas and telangiectasia.

BRaf is a protein kinase involved in the regulation of the mitogen activated protein kinase (MAPK) signaling pathway. Mutations in BRaf can induce constitutive signaling through the MAPK pathway which may result in uncontrolled cell proliferation. Use of BRaf inhibitors has been demonstrated to be associated with inhibition of MAPK signaling, as can be determined by reduction in levels of phosphorylated Extracellular Signal-Regulated Kinase (ERK), which is the downstream effector of BRaf. Yet, it has been observed that BRaf inhibitors can paradoxically induce an opposite effect of activating MAPK signaling in BRaf wild-type cells (as determined by increased levels of phosphorylated ERK). The underlying mechanisms of paradoxical MAPK activation have been attributed to dimerization of wild-type BRaf and c-Raf and transactivation of the non-inhibited Raf protein leading to subsequent MAPK pathway activation.

Notwithstanding the underlying mechanism(s) causing the cutaneous adverse reactions, these adverse reactions are a serious drawback of the treatment with EGFR, PI3K, MEK and /or HER dimerization inhibitors, and may lead to treatment discontinuation and/or poor patient compliance.

Carnahan J. et al. (Mol. Cancer Ther. 9(8) August 2010) found that selective and potent Raf inhibitors can paradoxically stimulate normal cell proliferation. A series of orally bioavailable kinase inhibitors disclosed by Smith A.L. et al., J. Med. Chem. 2009, 52, 6189-6192 showed potent biochemical activity. For example, Compound 1 of the series (C-1) showed significant potency (^{WT}B-Raf Ki = 1 nmol/L, V600EB-Raf Ki = 1 nmol/L, and C-Raf Ki = 0.3 nmol/L).

Carnahan et al. found that in cells with wild-type B-Raf and mutated K-ras, exposure to Raf inhibitors resulted in a dose-dependent and sustained paradoxical activation of mitogen-activated protein kinase (MAPK) signaling. Raf inhibition led to entry into the cell cycle and enhanced proliferation. This paradoxical activation of MAPK can be used for treating cutaneous adverse reactions induced by treatment with EGFR or PI3K inhibitors (see PCT/IL2017/050301 and PCT/IL2018/050836), and HER dimerization inhibitors (see Nami B et al. "The Effects of Pertuzumab and Its Combination with Trastuzumab on HER2 Homodimerization and Phosphorylation". Cancers (Basel). 2019;11(3):37; Nami B et al. "The Effects of Pertuzumab and Its Combination with Trastuzumab on HER2 Homodimerization and Phosphorylation". Cancers, 11(3), 375, 2019;which are incorporated herein by reference in their entireties).

There is still a need in the art for the development of therapeutic compounds, compositions, and methods of treatment, to help alleviate the aforementioned cutaneous adverse reactions associated with administration of EGFR inhibitors, PI3K inhibitors, MEK inhibitors, HER dimerization inhibitors or combinations thereof.

Provided herein are the compound of Formula IV and compositions comprising it. Also provided are methods of treating cutaneous adverse reactions, as defined in the claims, using the compound and compositions of the present disclosure.

### Compounds

In one embodiment, not forming part of the present invention, provided herein is a compound of Formula I: 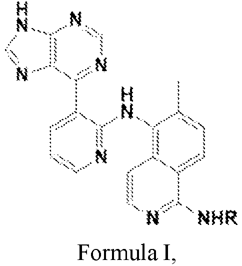 wherein R is selected from the group consisting of 3-ethynylphenyl, 3-chloro-4-fluorophenyl, 2-fluoro-4-iodophenyl, 4-chloro-3-(trifluoromethyl)phenyl, 3-(1,1-dimethylethyl)-1-methyl-1H-pyrazol-5-yl, 3-(trifluoromethoxy)phenyl, 3,5-dihydroxyphenyl or phenyl-3-sulfonamide, or a pharmaceutically acceptable salt or a solvate thereof.

In another embodiment, not forming part of the present invention, provided herein is a compound of Formula II: 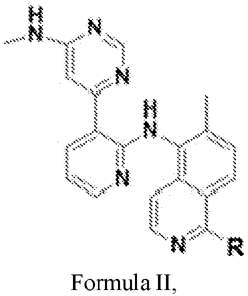 wherein R is NHR¹, wherein R¹ is 2-fluoro-4-iodophenyl, or a pharmaceutically acceptable salt or a solvate thereof.

In another embodiment, not forming part of the present invention, provided herein is a compound of Formula III: 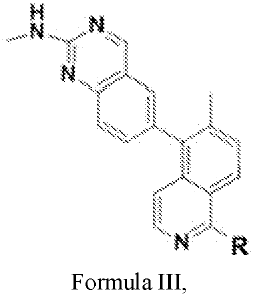 wherein R is NHR¹, wherein R¹ is 3-ethynylphenyl, 3-chloro-4-fluorophenyl, 2-fluoro-4-iodophenyl, or 4-chloro-3-(trifluoromethyl) phenyl, or a pharmaceutically acceptable salt or a solvate thereof.

In one embodiment, the compounds of present disclosure inhibit the activity of BRaf. In one embodiment, the compounds of present disclosure may have an IC50 towards BRaf of about 0.5x10⁻⁸ M to about 5x10⁻⁸ M, about 1x10⁻⁸ M to about 5x10⁻⁸ M, about 1x10⁻⁸ M to about 3.5x10⁻⁸ M, or about 1x10⁻⁸ M to about 3x10⁻⁸ M.

In one embodiment, the compounds of present disclosure increase the activity of Mitogen-Activated Protein Kinases (MAPK).

In one embodiment, the compounds of present disclosure increase the activity of MAPK and simultaneously inhibit the activity of BRaf.

In one embodiment, the activity of MAPK is determined by measuring the phosphorylation of Extracellular Signal-Regulated Kinase (ERK) and calculating a ratio of phospho-ERK to total ERK.

In one embodiment, the activity of MAPK is determined by measuring the paradoxical proliferation of MIA-PaCa-2 cell (see FIG. 1F)

In one embodiment, the compounds of the present disclosure increase the ratio of phospho-ERK to total ERK by at least about 1.025 fold, 1.05 fold, 1.10-fold, 1.15-fold, 1.20-fold, 1.25-fold, 1.30 fold, 1.35-fold, 1.40-fold, 1.45-fold, 1.5-fold, 1.6-fold, 1.7-fold, 1.8-fold, 2-fold, 2.25-fold, 2.5-fold, 2.75-fold, 3-fold, 3.25-fold, 3.5-fold, 3.75-fold, 4-fold, 4.25-fold, 4.5-fold, 4.75-fold, 5-fold, 5.25-fold, 5.50-fold, 5.75-fold, 6-fold, 6.25-fold, 6.50-fold, 6.75-fold, 7-fold, 7.25-fold, 7.5-fold, 8-fold, 8.5-fold, 9-fold, 9.5-fold, 10-fold, 15-fold, 20-fold, 25-fold, 30-fold, 40-fold, 50-fold, 75-fold, 100-fold, 150-fold, or by about 200-fold, including values and ranges therebetween, compared to untreated or control-treated cells.

In one embodiment, the compounds of the present disclosure increase the ratio of phospho-ERK to total ERK by about 1.5-fold to about 50-fold, about 1.5-fold to about 25-fold, 1.5-fold to about 20-fold, about 1.5-fold to about 15-fold, about 2.5-fold to about 15-fold, about 2.5-fold to about 10-fold, about 3-fold to about 20-fold, about 3-fold to about 15-fold, about 4-fold to about 20-fold, about 4-fold to about 15-fold, about 4-fold to about 10-fold, about 5-fold to about 20-fold, about 5-fold to about 15-fold, including values and ranges therebetween, compared to untreated or control-treated cells.

In one embodiment, the compounds of the present disclosure increase the level of phospho-ERK relative to total ERK by at least about 2.5%, 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 225%, 250%, 275%, 300%, 325%, 350%, 375% 400%, 425%, 450%, 475%, 500%, 550%, 600%, 650%, 700%, 750%, 800%, 850%, 900%, 950%, 1000%, 1100%, 1200%, 1300%, 1400%, 1500%, 1600%, 1700%, 1800%, 1900%, 2000%, 2250%, 2500%, 2750%, 3000%, 3250%, 3500%, 4000%, 4500%, 4750%, 5000%, 5500%, 6,000%, 6500%, 7,000%, 7500%, 8,000%, 9,000%, or 10,000%, including values and ranges therebetween, compared to untreated or control-treated cells.

In one embodiment, the compounds of present disclosure show no phototoxicity or low phototoxicity (have a PIF of less than 2). The level of phototoxicity can be determined by measuring a Photo-Irritation Factor (PIF) or a Mean Photo Effect (MPE).

In one embodiment, a PIF can be calculated using the following formula:
PIF = IC50(-Irr) / IC50(+Irr), where PIF > 5 indicates phototoxicity; 2< PIF <5 indicates probable phototoxicity; PIF < 2 indicates no phototoxicity. In one embodiment, the compounds of present disclosure have a PIF of less than 5. In another embodiment, the compounds of present disclosure have a PIF of less than 2.

In one embodiment, the MPE can be calculated by comparing the complete concentration-response curves. MPE is a weighted average of the difference in response of equivalent doses normalized by the shift in IC50. MPE > 0.15 indicates phototoxicity; 0.1<MPE<0.15 indicates probable phototoxicity; MPE <0.1 indicates no phototoxicity. In one embodiment, the compounds of present disclosure have a MPE of less than 0.15. In another embodiment, the compounds of present disclosure have a MPE of less than 0.1.

According to the present invention, the compound has the following Formula IV

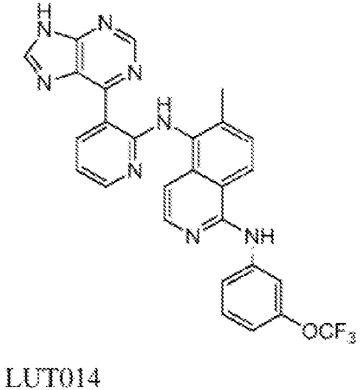

### Compositions

In some embodiments, provided herein are pharmaceutical compositions comprising a compound of Formula IV or a pharmaceutically acceptable salt or a solvate thereof, or a combination thereof, and a pharmaceutically acceptable carrier or excipient.

According to the invention, the pharmaceutical composition comprises 0.03 to 0.1 % w/w of the compound or a pharmaceutically acceptable salt or a solvate thereof, or a combination thereof, based on the total weight of the composition. For example, the pharmaceutical composition may comprise about 0.03, 0.04, 0.05, 0.06, 0.07, 0.08, 0.09, 0.1% w/w, including values and ranges therebetween, of any of the compounds disclosed herein or combinations thereof. In some embodiments, the pharmaceutical composition may comprise 0.03% 0.04%, 0.03% to 0.05%, 0.03% to 0.06%, 0.03% to 0.07%, 0.03% to 0.08%, 0.03% to 0.09%, 0.03% to 0.1% 0.04% to 0.05%, 0.04% to 0.06%, 0.04% to 0.07%, 0.04% to 0.08%, 0.04% to 0.09%, 0.04% to 0.1%, %, 0.05% to 0.06%, 0.05% to 0.07%, 0.05% to 0.08%, 0.05% to 0.09%, 0.05% to 0.1% weight/weight (w/w), including values and ranges therebetween, of any of the compound disclosed herein. For example, the pharmaceutical composition may comprise about 0.03, 0.04, 0.05, 0.06, 0.07, 0.08, 0.09, 0.1 % w/w, including values and ranges therebetween, of any of the compound disclosed herein or combinations thereof. In some embodiments, the pharmaceutical composition may comprise from 0.03% to 0.04%, 0.03% to 0.05%, 0.03% to 0.06%, 0.03% to 0.07%, 0.03% to 0.08%, 0.03% to 0.09%, 0.03% to 0.1% weight/weight (w/w), including values and ranges therebetween, of the compound disclosed herein.

According to the present invention, the pharmaceutical composition comprises 0.03 % w/w to 0.1% w/w of the compound of Formula IV or a pharmaceutically acceptable salt or a solvate thereof, or a combination thereof, based on the total weight of the composition. For example, the pharmaceutical composition may 0.03, 0.04, 0.05, 0.06, 0.07, 0.08, 0.09, 0.1% w/w, including values and ranges therebetween, of the compound disclosed herein or combinations thereof. In some embodiments, the pharmaceutical composition may comprise from 0.03% to 0.04%, 0.03% to 0.05%, 0.03% to 0.06%, 0.03% to 0.07%, 0.03% to 0.08%, 0.03% to 0.09%, 0.03% to 0.1%, 0.04% to 0.05%, 0.04% to 0.06%, 0.04% to 0.07%, 0.04% to 0.08%, 0.04% to 0.09%, 0.04% to 0.1%, %, 0.05% to 0.06%, 0.05% to 0.07%, 0.05% to 0.08%, 0.05% to 0.09%, 0.05% to 0.1% w/w, including values and ranges therebetween, of any of the compound disclosed herein.

According to the present invention, the pharmaceutical composition comprises 0.03% w/w to 0.1% w/w of the compound of Formula IV or a pharmaceutically acceptable salt or a solvate thereof, or a combination thereof, based on the total weight of the composition. For example, the pharmaceutical composition may comprise 0.03, 0.04, 0.05, 0.06, 0.07, 0.08, 0.09, 0.1% w/w, including values and ranges therebetween, of the compound disclosed herein or combinations thereof. In some embodiments, the pharmaceutical composition may comprise from 0.03% to 0.04%, 0.03% to 0.05%, 0.03% to 0.06%, 0.03% to 0.07%, 0.03% to 0.08%, 0.03% to 0.09%, 0.03% to 0.1%, 0.04% to 0.05%, 0.04% to 0.06%, 0.04% to 0.07%, 0.04% to 0.08%, 0.04% to 0.09%, 0.04% to 0.1%, 0.05% to 0.06%, 0.05% to 0.07% 0.05% to 0.08%, 0.05% to 0.09%, 0.05% to 0.1% w/w, including values and ranges therebetween, of any of the compound disclosed herein.

In one embodiment, the pharmaceutical composition comprising the compound disclosed herein is formulated for topical administration.

According to the present invention, provided herein is a topical pharmaceutical composition comprising a compound of Formula IV or a pharmaceutically acceptable salt or a solvate thereof, or a combination thereof, and a pharmaceutically acceptable carrier or excipient. Compositions for topical administration (topical compositions) can be in the form of a gel, a hydrogel, an ointment, a cream, a paste, a foam, a spray, a lotion, a liquid, or a dermal patch and may comprise any of the disclosed compound(s) in any of the amounts described herein.

The topical composition is a gel and comprises 0.03 % w/w to 0.1 % w/w of the compound of Formula IV or a pharmaceutically acceptable salt or a solvate thereof, or a combination thereof, and a pharmaceutically acceptable carrier or excipient.

In some embodiments, the topical composition is a gel and comprises 0.03% w/w of the compound of Formula IV or a pharmaceutically acceptable salt or a solvate thereof, or a combination thereof, and a pharmaceutically acceptable carrier or excipient.

Topical compositions useful in the present disclosure may be formulated as a solution. Such compositions may comprise an emollient preferably containing from about 1% to about 50% of an emollient(s). As used herein, the term "emollient" refers to materials used for the prevention or relief of dryness, as well as for the protection of the skin. A number of suitable emollients are known and may be used in the present disclosure. For example, Sagarin, Cosmetics, Science and Technology, 2nd Edition, Vol. 1, pp. 32-43 (1972) and the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook") contains numerous examples of suitable materials.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in Sagarin, Cosmetics, Science and Technology, 2nd Edition, Vol. 1, pp. 72-73 (1972) and the ICI Handbook pp. 1693-1697.

The topical compositions useful in the present disclosure may be formulated as emulsions. If the carrier for a topical composition is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, in McCutcheon's Detergents and Emulsifiers, North American Edition, pp. 317-324 (1986), and the ICI Handbook, pp. 1673-1686.

Lotions and creams can be formulated as emulsions. Such lotions may comprise from 0.5% to about 5% of an emulsifier(s). Creams may comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

The topical compositions of this disclosure can also be formulated as a gel (e.g., an aqueous, alcohol, alcohol/water, or oil gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils include, but are not limited to, hydrogenated butylene/ethylene/styrene copolymer and hydrogenated ethylene/propylene/styrene copolymer. Gel compositions may comprise between about 0.1% and 5%, by weight, of such gelling agents.

Another topical dosage form is a paste. Pastes are stiff semi-solid topical dosage forms, containing a high proportion of finely powdered solid such as starch, zinc oxide, calcium carbonate and talc. Another component of pastes is a base, which may be a hydrocarbon, an absorption ingredient, a water-miscible ingredient or a water-soluble ingredient. Pastes are less greasy than ointments.

In one embodiment, the topical compositions of this disclosure can also be formulated as a sprayvabie topical formulation. The compositions can be in a form suitable for application by spraying from an aerosol or pump spray container. The spray formulation can comprise the compound of the disclosure a solution or suspension in a vehicle. The vehicle can optionally contain a polymer or combination of polymers which, when sprayed on the surface of the skin, forms a film on the skin.

In addition to the above carriers and excipients, other emollients and surface active agents can be incorporated into the topical compositions, including glycerol trioleate, acetylated sucrose distearate, sorbitan trioleate, polyoxyethylene (1) monostearate, glycerol monooleate, sucrose distearate, polyethylene glycol (50) monostearate, octylphenoxypoly (ethyleneoxy) ethanol, decaglycerin penta-isostearate, sorbitan sesquioleate, hydroxylated lanolin, lanolin, triglyceryl diisostearate, polyoxyethylene (2) oleyl ether, calcium stearoyl-2-lactylate, methyl glucoside sesquistearate, sorbitan monopalmitate, methoxy polyethylene glycol-22/dodecyl glycol copolymer (Elfacos E200), polyethylene glycol-45/dodecyl glycol copolymer (Elfacos ST9), polyethylene glycol 400 distearate, and lanolin derived sterol extracts, glycol stearate and glycerol stearate; alcohols, such as cetyl alcohol and lanolin alcohol; myristates, such as isopropyl myristate; cetyl palmitate; cholesterol; stearic acid; propylene glycol; glycerin, sorbitol and the like.

The compound or compositions described herein can be used alone or in combination with other active agents. In some embodiments, the compounds or compositions for the treatment or prevention of a cutaneous adverse reaction, as defined in claims, of EGFR inhibitors, PI3K inhibitors, MEK inhibitors, HER dimerization inhibitors or combinations thereof are topically administered in a subject in need thereof without coadministration of another active agent. In some embodiments, the active agent of the composition provided herein consists of a compound having Formula IV.

### Methods

Provided herein are methods of treating, preventing, and/or ameliorating the dermatological or cutaneous adverse reactions, as defined in the claims, induced by chemotherapy agents inhibiting the pathway downstream to EGFR, such as EGFR inhibitors, PI3K inhibitors, MEK inhibitors, HER dimerization inhibitors or combinations thereof.

In some embodiments, not forming part of the present invention, provided herein is a method for treating, ameliorating, and/or preventing a cutaneous adverse reaction of EGFR inhibitors, PI3K inhibitors, MEK inhibitors, HER dimerization inhibitors or combinations thereof in a subject in need thereof, comprising administering a therapeutically effective amount of a composition comprising a compound of Formula I, Formula II, Formula III, or a combination thereof; and a pharmaceutically acceptable carrier or excipient.

In some embodiments, not forming part of the present invention, provided herein is a method for treating, ameliorating, and/or preventing EGFR inhibitors, PI3K inhibitors, MEK inhibitors, and/or HER dimerization inhibitors-induced acneiform lesions in a subject in need thereof, comprising administering a therapeutically effective amount of a composition comprising a compound of Formula I, Formula II, Formula III, or a combination thereof; and a pharmaceutically acceptable carrier or excipient.

According to the present invention, provided herein is a method for treating, ameliorating, and/or preventing a cutaneous adverse reaction, as defined in the claims, of EGFR inhibitors, PI3K inhibitors, MEK inhibitors, HER dimerization inhibitors or combinations thereof in a subject in need thereof, comprising administering a therapeutically effective amount of a composition comprising a compound of Formula IV; and a pharmaceutically acceptable carrier or excipient.

According to the present invention, provided herein is a method for treating, ameliorating, and/or preventing EGFR inhibitors, PI3K inhibitors, MEK inhibitors, and/or HER dimerization inhibitors-induced acneiform lesions in a subject in need thereof, comprising administering a therapeutically effective amount of a composition comprising a compound of Formula IV; and a pharmaceutically acceptable carrier or excipient.

In some embodiments, not forming part of the present invention, methods for treating, ameliorating, and/or preventing a cutaneous adverse reaction of EGFR inhibitors, PI3K inhibitors, MEK inhibitors, HER dimerization inhibitors or combinations thereof in a subject in need thereof comprise administering topically a therapeutically effective amount of a topical pharmaceutical composition comprising a compound of Formula I, Formula II, Formula III, or a combination thereof, or a pharmaceutically acceptable salt or a solvate thereof, or a combination thereof; and a pharmaceutically acceptable carrier or excipient.

Accoring to the present invention, methods for treating, ameliorating, and/or preventing a cutaneous adverse reaction, as defined in the claims, of EGFR inhibitors, PI3K inhibitors, MEK inhibitors, HER dimerization inhibitors or combinations thereof in a subject in need thereof comprise administering topically a therapeutically effective amount of a topical pharmaceutical composition comprising a compound of Formula IV, or a pharmaceutically acceptable salt or a solvate thereof, or a combination thereof; and a pharmaceutically acceptable carrier or excipient.

In some embodiments, not forming part of the present invention, methods for treating, ameliorating, and/or preventing a cutaneous adverse reaction of EGFR inhibitors, PI3K inhibitors, MEK inhibitors, HER dimerization inhibitors or combinations thereof in a subject in need thereof comprise administering a therapeutically effective amount of a composition comprising a combination of any of the compounds disclosed herein and a pharmaceutically acceptable carrier or excipient.

According to the present invention, methods for treating, ameliorating, and/or preventing a cutaneous adverse reaction, as defined in the claims, of EGFR inhibitors, PI3K inhibitors, MEK inhibitors, HER dimerization inhibitors or combinations thereof in a subject in need thereof comprise administering a therapeutically effective amount of a composition comprising a compound of formula 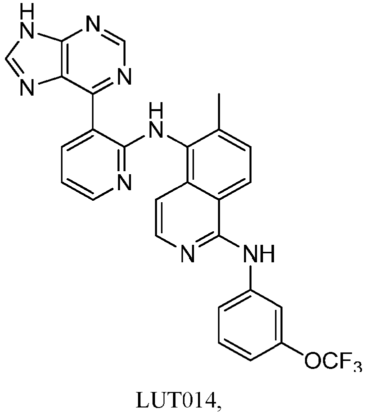 in any of the w/w% amounts disclosed herein and a pharmaceutically acceptable carrier or excipient.

Dermatological or cutaneous adverse reactions induced by anti-cancer treatment agents such as EGFR inhibitors, PI3K inhibitors, MEK inhibitors, HER dimerization inhibitors or combinations thereof include acneiform lesions (also referred herein as acneifrom rash).

In some embodiments, the subject is a mammal, human or non-human. In some embodiments, the subject is a human.

In one embodiment, the subject is receiving an EGFR inhibitor, PI3K inhibitor, MEK inhibitor, HER dimerization inhibitor or a combination thereof at the time of the administration of the compound/composition of the present disclosure. In some embodiments, the subject has melanoma, lung cancer, head & neck cancer, colorectal cancer, brain cancer, multiple myeloma, breast cancer, pancreatic cancer, liver cancer and neurofibromatosis.

In another embodiment, the compound/composition of the present disclosure are administered to the subject prior to or after administration of an EGFR inhibitor, PI3K inhibitor, MEK inhibitor, HER dimerization inhibitors or a combination thereof.

In another embodiment, the compound/composition of the present disclosure are administered to the subject prior to or after appearance of the cutaneous reactions.

In some embodiments, methods disclosed herein comprise topical administration of a therapeutically effective amount of the compound/composition of the present disclosure.

Methods comprising topical administration comprise local administration or application of any of the compositions disclosed herein to the skin of the subject. In some embodiments, topical administration comprises topically administering a composition, wherein the composition is a gel, a hydrogel, an ointment, a cream, a spray, a dermal patch, a paste, a foam, a lotion or a liquid.

In some embodiments, the amount of the compound administered depends on the nature of the compound, the formulation, and/or the severity of the cutaneous reaction. The therapeutically effective amount that is administered to a patient can be determined by dose-ranging clinical studies known in the art.

In some embodiments, the dosage regimen and/or length of treatment depends on the nature of the compound, the formulation, and/or the severity of the cutaneous reaction.

According to some embodiments, the method comprises topically administering, once daily, to a skin area affected by the EGFR inhibitors, PI3K inhibitors MEK and/or HER dimerization - inhibitors induced of a subject in need thereof the topical pharmaceutical composition. In some embodiments, the method comprises topically administering, once daily, to a skin area affected by the EGFR inhibitors-induced cutaneous lesions the topical pharmaceutical composition.

In some embodiments, the subject in need thereof is treated with EGFR inhibitors. In some embodiments, the subject in need thereof is treated with PI3K inhibitors. In some embodiments, the subject in need thereof is treated with MEK inhibitors. In some embodiments, the subject in need thereof is treated with EGFR inhibitors, PI3K inhibitors, MEK inhibitors and/or HER dimerization inhibitors. In some embodiment, is a subject having cancer and treated with EGFR inhibitors.

In some embodiments, the subject in need thereof has grade 1 acneiform lesions. In some embodiments, the subject in need thereof has grade 2 acneiform lesions. In some embodiments, the subject in need thereof has grade 3 acneiform lesions. In some embodiments, the subject in need thereof has grade 4 acneiform lesions.

In some embodiments, the topical pharmaceutical composition is administered once daily for 2 weeks, 3 weeks, 4 weeks, 5 weeks, 6 weeks, etc. In some embodiments, the topical composition is administered once daily for 4 to 6 weeks.

In some embodiments, the topical pharmaceutical composition is administered twice daily for 2 weeks, 3 weeks, 4 weeks, 5 weeks, 6 weeks, etc. In some embodiments, the topical pharmaceutical composition is administered twice daily for 4 to 6 weeks.

In some embodiments, the method comprises topically administering, once daily, to a skin area affected by the cutaneous lesions the topical pharmaceutical composition comprising from 0.03% to 0.1% w/w, of the compound of Formula IV or a pharmaceutically acceptable salt or a solvate thereof, or a combination thereof, and a pharmaceutically acceptable carrier or excipient. In some embodiments, the topical pharmaceutical composition is administered once daily for 4 to 6 weeks.

In some embodiments, the method comprises topically administering, twice daily, to a skin area affected by the cutaneous lesions the topical pharmaceutical composition comprising from 0.03% to 0.1% w/w of the compound of Formula IV or a pharmaceutically acceptable salt or a solvate thereof, or a combination thereof, and a pharmaceutically acceptable carrier or excipient. In some embodiments, the topical pharmaceutical composition is administered twice daily for 4 to 6 weeks.

In some embodiments, the methods reduce the severity of the cutaneous adverse reactions, for example as using the NCI CTCAE version 5.0 skin and subcutaneous tissue disorders grading scale. For example, the methods reduce the severity of the cutaneous adverse lesions from grade 2 to grade 1. In some embodiments, the methods prevent the escalation of the acneiform lesions. In some embodiments, the methods reduces the severity of the acneiform lesions from grade 4 to grade 3, 2, 1, or 0. the method reduces the severity of the acneiform lesions from grade 3 to grade 2, 1, or 0. the method reduces the severity of the acneiform lesions from grade 3 to grade 2, 1, or 0. the method reduces the severity of the acneiform lesions from grade 2 to grade 1, or 0. the method reduces the severity of the acneiform lesions from grade 1 to grade 0.

In some embodiments, the administration results in a significant reduction in severity of the cutaneous adverse reaction as early as 1 week after the initial administration of the topical pharmaceutical composition. In some embodiments, the methods reduce the severity of the cutaneous adverse reactions, for example as using the NCI CTCAE version 5.0 skin and subcutaneous tissue disorders grading scale. In some embodiments, the methods reduce the severity of the cutaneous adverse lesions from grade 2 to grade 1.

In some embodiments, the method provides sustained benefit for at least 7 days, 14 days, 21 days 28 days, 35 days, 42 days, 49 days, 56 days, or more after administration had terminated.

In some embodiments, the method prevents the apparition of EGFR inhibitors, PI3K inhibitors, MEK inhibitors and/or HER dimerization inhibitors-induced cutaneous lesions when administered concomitantly with the EGFR inhibitors, PI3K inhibitors, MEK- inhibitors and/or HER dimerization inhibitors.

In some embodiments of the methods disclosed herein, an EGFR inhibitor is selected from Iressa^{®} (gefitinib), Tarceva^{®} (erlotinib), Tykerb^{®} (Lapatinib), Erbitux^{®} (cetuximab), Vectibix^{®} (panitumumab), Caprelsa^{®} (vandetanib), Portrazza^{®} (necitumumab), Tagrisso^{®} (osimertinib), Gilotrif^{®} (afatinib), nerlunx^{®} (neratinib), and combinations thereof.

In some embodiments of the methods disclosed herein, a PI3K inhibitor is selected from GDC-0980 (Apitolisib), GDC-0941 (Pictilisib), BAY 80-6946 (Copanlisib), BKM120 (Puparlisib), NVP-BEZ235 (Dactolisib), IPI 145 (Duvelisib), Idelalisib (GS-1101 or CAL-101), wortmannin, LY294002 and combinations thereof.

In some embodiments of the methods disclosed herein, a MEK inhibitor is selected from Trametinib (GSK1120212), Cobimetinib (XL518), Binimetinib (MEK162), Selumetinib, PD-325901, CI-1040, PD035901, UO126, TAK-733, and combinations thereof.

In some embodiments of the methods disclosed herein, a HER dimerization inhibitor is pertuzumab (Perjeta).

In some embodiments, the methods disclosed herein reduce the severity of the cutaneous adverse reactions, i.e. acneiform lesions.

Success of treating cutaneous adverse reactions can be measured using methods known in the art.

The most validated, commonly used and FDA approved system to grade the severity of cutaneous adverse reactions is National Cancer Institute's Common Terminology Criteria for Adverse Events (CTCAE) version 5.0, which recognizes 4 grades shown in Table 1 below.

**Table 1**

| **NCI-CTCAE version 5.0 grading scale of skin and subcutaneous tissue disorders** | |
|---|---|
| Grade 1 | Papules and/or pustules covering <10% of the BSA which may or may not be associated with symptoms of pruritus or tenderness |
| Grade 2 | Papules and/or pustules covering 10-30% of the BSA which may or may not be associated with symptoms of pruritus or tenderness; associated with psychosocial impact; limiting instrumental ADL; papules and/or pustules covering > 30% BSA with or without mild symptoms |
| Grade 3 | Papules and/or pustules covering >30% BSA with moderate or severe symptoms; limiting self-care ADL, associated with local superinfection with oral antibiotics indicated |
| Grade 4 | Life-threatening consequences; papules and/or pustules covering any % BSA, which may or may not be associated with symptoms of pruritus or tenderness and are associated with extensive superinfection with IV antibiotics indicated |
| Grade 5- Death | |
| | |
| BSA = Body surface area; ADL = activity of daily living | |

In one embodiment, the methods disclosed herein reduce the severity of the cutaneous adverse reactions from grade 4 to grade 3, 2, 1, or 0, as defined by National Cancer Institute Common Terminology Criteria for Adverse Events (NCI-CTCAE) version 5.0.

In one embodiment, the methods disclosed herein reduce the severity of the cutaneous adverse reactions from grade 3 to grade 2, 1, or 0, as defined by NCI-CTCAE version 5.0.

In one embodiment, the methods disclosed herein reduce the severity of the cutaneous adverse reactions from grade 2 to grade 1 or 0, as defined by NCI-CTCAE version 5.0.

In one embodiment, the methods disclosed herein reduce the severity of the cutaneous adverse reactions from grade 1 to grade 0, as defined by NCI-CTCAE version 5.0.

In one embodiment, the methods disclosed herein prevent, partially or completely, the development of cutaneous adverse reactions.

In one embodiment, the methods disclosed herein prevent, partially or completely, the development of grade 4, grade 3, grade 2, or grade 1 of the cutaneous adverse reactions, as defined by NCI-CTCAE version 5.0.

In one embodiment, the methods disclosed herein prevent the escalation of the cutaneous adverse reaction. For example, in one embodiment, the methods disclosed herein prevent the escalation of the cutaneous adverse reaction from grade 0 to grade 1, 2, 3, or 4, as defined by NCI-CTCAE version 5.0. In another embodiment, the methods disclosed herein prevent the escalation of the cutaneous adverse reaction from grade 1 to grade 2, 3, or 4, as defined by NCI-CTCAE version 5.0. In another embodiment, the methods disclosed herein prevent the escalation of the cutaneous adverse reaction from grade 2 to grade 3 or 4, as defined by NCI-CTCAE version 5.0. In another embodiment, the methods disclosed herein prevent the escalation of the cutaneous adverse reaction from grade 3 to grade 4, as defined by NCI-CTCAE version 5.0.

Another system that may be used to grade the severity of cutaneous adverse reactions is Lacouture grading scale shown in Table 2 below.

**Table 2**

| **Adverse Event** | **Grade 1** | | **Grade 2** | | **Grade 3** | | **Grade 4** |
|---|---|---|---|---|---|---|---|
| Papulopustular eruption Grading individually for face, scalp, chest or back) | 1A Papules or pustules <5 OR 1 area of erythema or edema <1 cm in size | 1B Papules or pustules <5; OR 1 area of erythema or edema <1 cm in size AND pain or pruritus | 2A Papules or pustules 6-20; OR 2-5 areas of erythema or edema <1cm in size | 2B Papules or pustules 6-20; OR 2-5 areas of erythema or edema <1cm in size AND pain, pruritus, or effect on emotions or functioning | 3A Papules or pustules >20; OR more than 5 areas of erythema or edema <1cm in size | 3B Papules or pustules >20; OR more than 5 areas of erythema or edema <1cm in size AND pain, pruritus, or effect on emotions or functioning | |
| Nail changes-Nail Plate | Onycholysis or ridging without pain | | Onycholysis with mild/moderate pain; any nail plate lesion interfering with instrumental ADL | | Nail plate changes interfering with self-care ADL | | |
| Nail changes-Nail fold | Disruption or absence of cuticle; OR erythema | | Erythematous/tender/painful; OR pyogenic granuloma; OR crusted lesions OR any fold lesion interfering on instrumental ADL | | Periungual abscess: OR fold changes interfering with self-care ADL | | |
| Nail changes-Digit tip | Xerosis AND/OR erythema without pain | | Xerosis AND/OR erythema with mild/moderate pain or stinging; OR fingertip fissures; OR any digit tip lesion interfering with instrumental ADL | | Digit tip lesions interfering with self-care ADL | | |
| Erythema | Painless erythema, blanching; erythema covering <10% BSA | | Painful erythema, blanching; erythema covering 10-30% BSA | | Painful erythema, nonblanching; erythema covering >30% BSA | | |

| **Adverse Event** | **Grade 1** | **Grade 2** | | **Grade 3** | | **Grade 4** |
|---|---|---|---|---|---|---|
| Pruritus | Mild OR localized, intermittent, not requiring therapy. | 2A Moderate localized OR widespread intermittent AND Requiring intervention | 2B Moderate localized OR widespread constant AND Requiring intervention | Severe, widespread constant AND interfering with sleep | | |
| Xerosis | Scaling/flaking covering <10% BSA NO erythema/pruritus/effect on emotions or functioning | 2A Scaling/flaking covering 10-30% BSA + pruritus OR effect on emotions/ functioning | 2B Scaling/flaking + pruritus covering 10-30% BSA AND effect on emotions/ functioning + erythema | 3A Scaling/flaking covering >30% BSA AND pruritus AND erythema AND effect on emotions/ functioning AND fissuring/cracking + fissuring/ cracking | 3B Scaling/flaking covering >30% BSA AND pruritus AND erythema AND effect on emotions/ functioning AND fissuring/cracking + signs of super infection | |

| **Adverse Event** | **Grade 1** | **Grade 2** | | **Grade 3** | **Grade 4** |
|---|---|---|---|---|---|
| Hair changes; Scalp hair loss or alopecia | Terminal hair loss <50% of normal for that individual that may or may not be noticeable to others but is associated with increased shedding and overall feeling of less volume. May require different hair style to cover but does not require hairpiece to camouflage | 2A: Hair loss associated with marked increase in shedding and 50%-74% loss compared to normal for that individual. | 2B: Marked loss of at least 75% hair compared to normal for that individual with inability to camouflage except with a full wig OR new cicatricial hair loss documented by biopsy that covers at least 5% scalp surface area. | | |
| | | Hair loss is apparent to others, may be difficult to camouflage with change in hair style and may require hairpiece. | May impact on functioning in social, personal or professional situations. | | |

| **Adverse Event** | **Grade 1** | **Grade 2** | | **Grade 3** | **Grade 4** |
|---|---|---|---|---|---|
| Hair Changes: disruption of normal hair growth (specify): | Some distortion of hair growth but does not cause symptoms or require intervention. | 2A: Distortion of hair growth in many hairs in a given area that cause discomfort or symptoms that may require individual hairs to be removed. | 2B: Distortion of hair growth of most hairs in a given area with symptoms or resultant problems requiring removal of multiple hairs. | | |
| - Facial hair (diffuse, not just in male beard/mustache areas) | | | | | |
| -Eyelashes | | | | | |
| -Eyebrows | | | | | |
| -Body Hair | | | | | |
| -Beard and moustache hair | | | | | |

| **Adverse Event** | **Grade 1** | **Grade 2** | | **Grade 3** | **Grade 4** |
|---|---|---|---|---|---|
| Hair Changes: increased hair changes (specify): | Increase in length, thickness and/or density of hair that the patient is able to camouflage by periodic shaving, bleaching or removal of individual hairs. | 2A: Increase in length, thickness and/or density of hairs that is very noticeable and requires regular shaving or removal of hairs in order to camouflage. | 2B: Marked increase in hair density, thickness and/or length of hair that requires either frequent shaving or destruction of the hair to camouflage. | | |
| -Facial hair (diffuse, not just in male beard/mustache areas) | | | | | |
| -Eyelashes | | | | | |
| -Eyebrows | | | | | |
| -Body Hair | | | | | |
| -Beard and moustache hair (hirsutism) | | | | | |
| | | | May cause symptoms related to hair overgrowth. | | |
| | | May cause mild symptoms related to hair overgrowth. | | | |
| | | | Without hair removal, inability to function normally in social, personal or professional situations. | | |

| **Adverse Event** | **Grade 1** | | **Grade 2** | | **Grade 3** | | **Grade 4** |
|---|---|---|---|---|---|---|---|
| Flushing | 1A. Face OR chest, asymptomatic, transient | 1B. Any location, asymptomatic, permanent | 2A. Symptomatic on face, or chest, transient | 2B. Symptomatic on face, or chest, permanent | 3A. Face and chest, transient, symptomatic | 2B. Face and chest, permanent, symptomatic | |
| Telanglectasia | One area (<1cm diameter) NOT affecting emotions or functioning | | 2A 2-5 (<1cm diameter) areas NOT affecting emotions or functioning | 2B 2-5 (<1cm diameter) areas affecting emotions or functioning | More than 6 (<1cm diameter) OR confluent areas affecting emotions or functioning | | |
| Hyperpigmentation | One area (<1cm diameter) NOT affecting emotions or functioning | | 2A 2-5 (<1cm diameter) areas NOT affecting emotions or functioning | 2B 2-5 (<1cm diameter) areas affecting emotions or functioning | More than 6 (<1cm diameter) OR confluent areas affecting emotions or functioning | | |

| **Adverse Event** | **Grade 1** | **Grade 2** | **Grade 3** | **Grade 4** |
|---|---|---|---|---|
| Mucositis | Mild erythema or edema, and asymptomatic | Symptomatic (mild pain, opioid not required); erythema or limited ulceration, can eat solid foods and take oral medication (Oral mucositis only) | Pain requiring opioid analgesic; erythema and ulceration, cannot eat solids, can swallow liquids (Oral mucositis only) | erythema and ulceration, cannot tolerate PO intake; require tube feeding or hospitalization (Oral mucositis only) |
| -Oral | | | | |
| -Anal | | | | |
| Radiation dermatitis | Faint erythema or dry desquamation | Moderate to brisk erythema; patchy moist desquamation, mostly confined to skin folds and creases; moderate edema | Moist desquamation other than skin folds and creases; bleeding induced by minor trauma or abrasion | Skin necrosis or ulceration of full thickness dermis; spontaneous bleeding from involved site |
| Hyposalivation | Can eat but requires liquids, no effect on speech | Moderate/thickened saliva: cannot eat dry foods, mild speech impairment (sticky tongue, lips, affecting speech) | No saliva, unable to speak without water, no oral intake without water | |
| Taste | Altered or reduced taste; no impact on oral intake | Altered or reduced taste affecting interest and ability to eat no intervention required | Taste abnormalities, requires intervention | |

In one embodiment, the methods disclosed herein reduce the severity of the cutaneous adverse reactions from grade 4 to grade 3B, 3A, 2B, 2A, 1B, or 1A, as defined by Lacouture grading scale.

In one embodiment, the methods disclosed herein reduce the severity of the cutaneous adverse reactions from grade 3B to grade 3A, 2B, 2A, 1B, or 1A, as defined by Lacouture grading scale.

In one embodiment, the methods disclosed herein reduce the severity of the cutaneous adverse reactions from grade 3A to grade 2B, 2A, 1B, or 1A, as defined by Lacouture grading scale.

In one embodiment, the methods disclosed herein reduce the severity of the cutaneous adverse reactions from grade 2B to grade 2A, 1B, or 1A, as defined by Lacouture grading scale.

In one embodiment, the methods disclosed herein reduce the severity of the cutaneous adverse reactions from grade 2A to grade 1B or 1A, as defined by Lacouture grading scale.

In one embodiment, the methods disclosed herein reduce the severity of the cutaneous adverse reactions from grade 1B to grade 1A, as defined by Lacouture grading scale.

In one embodiment, the methods disclosed herein prevent, partially or completely, the development of grade 4, grade 3B, grade 3A, grade 2B, grade 2A, grade 1B, or grade 1A of the cutaneous adverse reactions, as defined by Lacouture grading scale.

In one embodiment, the methods disclosed herein prevent the escalation of the cutaneous adverse reaction from grade 1A to grade 1B, 2A, 2B, 3A, 3B or 4, as defined by Lacouture grading scale. In another embodiment, the methods disclosed herein prevent the escalation of the cutaneous adverse reaction from grade 1B to grade 2A, 2B, 3A, 3B or 4, as defined by Lacouture grading scale. In one embodiment, the methods disclosed herein prevent the escalation of the cutaneous adverse reaction from grade 2A to grade 2B, 3A, 3B or 4, as defined by Lacouture grading scale. In one embodiment, the methods disclosed herein prevent the escalation of the cutaneous adverse reaction from grade 2B to grade 3A, 3B or 4, as defined by Lacouture grading scale. In one embodiment, the methods disclosed herein prevent the escalation of the cutaneous adverse reaction from grade 3A to grade 3B or 4, as defined by Lacouture grading scale. In one embodiment, the methods disclosed herein prevent the escalation of the cutaneous adverse reaction from grade 3B to grade 4, as defined by Lacouture grading scale.

In some embodiments, the Patient self-reported quality of life FACT-EGFRI-13 questionnaire may be used to grade the severity of cutaneous adverse reactions shown in Table 3 below.

**Table 3-FACT-EGFRI-13 questionnaire**

| Below is a list of statements that other people with your illness have said are important. Please circle or mark one number per line to indicate your response as it applies to the past 7 days. | | | | | | |
|---|---|---|---|---|---|---|
| | | Not at all | A little bit | Some -what | Quite a bit | Very much |
| ST4 | My skin or scalp feels irritated | 0 | 1 | 2 | 3 | 4 |
| ST5 | My skin or scallop is dry or "flaky" | 0 | 1 | 2 | 3 | 4 |
| ST6 | My skin or scalp itches | 0 | 1 | 2 | 3 | 4 |
| ST7 | My skin bleeds easily | 0 | 1 | 2 | 3 | 4 |
| ST9 | I am bothered by a change in my skin's sensitivity to the sun | 0 | 1 | 2 | 3 | 4 |
| ST32 | My skin condition interferes with my ability to sleep | 0 | 1 | 2 | 3 | 4 |
| ST22 | My skin condition affects my mood | 0 | 1 | 2 | 3 | 4 |
| ST17 | My skin condition interferes with my social life | 0 | 1 | 2 | 3 | 4 |
| ST24 | I am embarrassed by my skin condition | 0 | 1 | 2 | 3 | 4 |
| ST37 | I avoid going out in public because of how my skin looks | 0 | 1 | 2 | 3 | 4 |
| ST26 | I feel unattractive because of how my skin looks | 0 | 1 | 2 | 3 | 4 |
| ST34 | Changes in my skin condition make daily life difficult | 0 | 1 | 2 | 3 | 4 |
| ST38 | The skin side effects from treatment have interfered with household tasks | 0 | 1 | 2 | 3 | 4 |
| ST16 | My eyes are dry | 0 | 1 | 2 | 3 | 4 |
| ST15 | I am bothered by sensitivity around my fingernails or toenails | 0 | 1 | 2 | 3 | 4 |
| ST29 | Sensitivity around by fingernails makes it difficult to perform household tasks | 0 | 1 | 2 | 3 | 4 |
| B5 | I am bothered by hair loss | 0 | 1 | 2 | 3 | 4 |
| ST11 | I am bothered by increased facial hair | 0 | 1 | 2 | 3 | 4 |

In some embodiments, there is provided the use of the topical pharmaceutical composition of this disclosure comprising a therapeutically and/or prophylactically effective dose of at compound described herein and a pharmaceutically acceptable carrier or excipient for the treatment, prevention or alleviation of EGFR inhibitors, PI3K inhibitors and/or MEK inhibitors-induced cutaneous, as defined in claims, by topical administration to a subject in need thereof, wherein said composition is formulated for topical administration.

In some embodiments, a method of treating and/or preventing EGFR inhibitor, PI3K inhibitor, MEK inhibitor and/or HER dimerization inhibitor -induced acneiform lesions in a subject in need thereof is provided, the method comprising administering once a day for 4 to 6 weeks to the skin of the subject a topical pharmaceutical composition comprising from 0.01 % weight/weight to 0.3 % weight/weight of a compound of Formula IV and a pharmaceutically acceptable carrier or excipient to the subject for treating EGFR inhibitor, PI3K inhibitor, MEK inhibitor and/or HER dimerization inhibitor-induced acneiform lesions. In some embodiments, the topical pharmaceutical composition is applied for four to six weeks to the skin of the subject in need thereof.

In some embodiments, the topical composition comprises from 0.03 % to 0.25 % weight/weight of the compound of Formula IV. In some embodiments, the topical composition comprises 0.03 % weight/weight of the compound of Formula IV

In some embodiments, the topical pharmaceutical composition is in the form of a gel, a hydrogel, an ointment, a cream, a spray, a dermal patch, a foam, a lotion or a liquid. In some embodiments, the topical pharmaceutical composition is in the form of a gel. In some embodiments, the topical pharmaceutical composition is in the form of a spray.

In some embodiments, the acneiform lesions are induced by an EGFR inhibitor. In some embodiments, the subject in need thereof is a subject has a cancer treated with EGFR inhibitor, PI3K inhibitor, MEK inhibitor and/or HER dimerization inhibitor.

In some embodiments, the subject in need thereof has grade 1 acneiform lesions. In some embodiments, the subject in need thereof has grade 2 acneiform lesions. In some embodiments, the subject in need thereof has grade 3 acneiform lesions. In some embodiments, the subject in need thereof has grade 4 acneiform lesions.

In some embodiments, the method reduces the severity or prevents escalation of the acneiform lesions. In some embodiments, the method reduces the severity of the acneiform lesions from grade 4 to grade 3, 2, 1, or 0. In some embodiments, the method reduces the severity of the acneiform lesions from grade 3 to grade 2, 1, or 0. In some embodiments, the method reduces the severity of the acneiform lesions from grade 2 to grade 1, or 0. In some embodiments, the method reduces the severity of the acneiform lesions from grade 1 to grade 0.

In some embodiments, the acneiform lesions are induced by treatment with gefitinib, erlotinib, lapatinib, cetuximab, panitumumab, vandetanib, necitumumab, osimertinib, afatinib, neratinib, pertuzumab or combinations thereof.

In some embodiments, compositions for use in the treatment and/or prevention of EGFR inhibitors, PI3K inhibitors, MEK inhibitors and/or HER dimerization inhibitors- induced cutaneous lesions, as defined in the claims, are provided, wherein the composition is topically administered to the skin of a subject in need thereof on a daily basis for 4 to 6 weeks, and the composition comprises from 0.03% to 0.1% w/w of the compound of Formula IV.

In some embodiments, compositions for use in the treatment and/or prevention of EGFR inhibitors, PI3K inhibitors, MEK inhibitors and/or HER dimerization inhibitors induced cutaneous lesions, as defined in the claims, are provided, wherein the composition is topically administered to the skin of a subject in need thereof twice daily for 4 to 6 weeks, and the composition comprises from 0.03% to 0.1% w/w of the compound of Formula IV. In some embodiments, the acneiform lesions are induced by an EGFR inhibitor. In some embodiments, the acneiform lesions are induced by treatment with gefitinib, erlotinib, lapatinib, cetuximab, panitumumab, vandetanib, necitumumab, osimertinib, afatinib, neratinib, pertuzumab or combinations thereof.

In some embodiments, the subject in need thereof is a subject has a cancer treated with EGFR inhibitor, PI3K inhibitor, MEK inhibitor and/or HER dimerization inhibitor.

In some embodiments, the subject in need thereof has grade 1 acneiform lesions. In some embodiments, the subject in need thereof has grade 2 acneiform lesions. In some embodiments, the subject in need thereof has grade 3 acneiform lesions. In some embodiments, the subject in need thereof has grade 4 acneiform lesions.

In some embodiments, the composition reduces the severity or prevents escalation of the acneiform lesions. In some embodiments, the composition reduces the severity of the acneiform lesions from grade 4 to grade 3, 2, 1, or 0. In some embodiments, the method reduces the severity of the acneiform lesions from grade 3 to grade 2, 1, or 0. In some embodiments, the composition reduces the severity of the acneiform lesions from grade 2 to grade 1, or 0. In some embodiments, the composition reduces the severity of the acneiform lesions from grade 1 to grade 0.

### EXAMPLES

The following examples illustrate certain embodiments of the invention but are not meant to limit the scope of the claims in any way. The following examples are put forth so as to provide those of ordinary skill in the art with a complete disclosure and description of how to make and use the described invention and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1: Reduction of skin toxicities induced by EGFR inhibitors with topical BRAF inhibitor therapy

Treatment of cancer with epidermal growth factor receptor (EGFR) inhibitors is limited by on-target skin toxicities induced by inhibition of the mitogen-activated protein kinase (MAPK) pathway. BRAF inhibitors are known to paradoxically activate the MAPK downstream of EGFR, which was confirmed using human skin keratinocytes. A phase 1 clinical trial was conducted testing the hypothesis that topical therapy with the BRAF inhibitor LUT014 could improve skin toxicities induced by EGFR inhibitors. Ten patients with metastatic colorectal cancer who had developed acneiform rash while being treated with cetuximab or panitumumab were enrolled in three cohorts. LUT014 was well tolerated and there were no dose-limiting toxicities. Improvement of acneiform rash in all grade 2 patients (six patients) treated in the low and intermediate cohorts was shown. As such, topical administration of LUT014 is safe and efficacious in improving rash from EGFR inhibitors, consistent with the mechanism of action inducting paradoxical MAPK activation.

Studies have reported that 75% to 90% of patients treated with EGFR inhibitor therapy experience some form of papulopustular, acneiform rash, which frequently leads to impaired quality of life and suboptimal anti-cancer treatment due to treatment interruptions, dose reductions or permanent discontinuation of EGFR inhibitor therapy. Inhibition of the activated EGFR in normal epithelial tissues results in inhibition of extracellular signal-regulated kinase 1/2 phosphorylation (pERK) and decreased keratinocyte proliferation and migration, and premature differentiation (Woodworth, C.D., et al., Mol Cancer Ther 4, 650-658 (2005)), with increases in chemokines attracting proinflammatory cells that trigger the resulting acneiform skin rash **(****Fig. 1A****,** see Pastore, S., et al. J Immunol 174, 5047-5056, 2005). However, EGFR inhibitor-induced acneiform rash is markedly reduced or does not develop in patients who receive a combination of an EGFR inhibitor with a systemic BRAF inhibitor for the treatment of *BRAF*^{V600} mutated colorectal carcinomas (Yaeger et al., Clin Cancer Res 21, 1313-1320, 2015). The decrease in toxicities with the combination is attributed to the paradoxical activation of the MAPK pathway induced by the BRAF inhibitor offsetting the decrease in pERK induced by the EGFR inhibitor (Hall-Jackson, et al. Chem Biol 6, 559-568, 1999; Su, et al. The New England journal of medicine 366, 207-215, 2012; Poulikakos, et al., Nature 464, 427-430, 2010; Hatzivassiliou, et al., Nature 464, 431-435, 2010; Heidorn, et al., Cell 140, 209-22, 2010; Halaban, et al., Pigment Cell Melanoma Res 23, 190-200, 2010). Paradoxical MAPK activation with a BRAF inhibitor refers to the increased MAPK pathway output, measured by increase in pERK and cell proliferation, when exposing cells that are wild type for *BRAF* to a BRAF inhibitor, in particular if there is strong upstream receptor tyrosine kinase or Ras activation (Solit, et al., Cancer Discov 4, 27-30, 2014; Holderfield, et al., Br J Cancer 111, 640-645, 2014). Based on this mechanistic understanding, it was hypothesized that a topical therapy with a BRAF inhibitor could reduce the severity of dose-limiting acneiform lesions associated with EGFR inhibitor treatment **(****Fig. 1B****).** The topical BRAF inhibitor would reverse the pERK inhibition through the induction of paradoxical MAPK activation in skin cells with wild-type *BRAF* leading to reactivation of the MAPK pathway (Escuin-Ordinas, et al., Nat Commun 7, 12348, 2016), and it would avoid interference with the anti-cancer treatment and other toxicities seen with systemic BRAF inhibitors.

LUT014 is a small molecule inhibitor of the serine/threonine-protein kinase BRAF **(****Fig. 1C****).** Culturing primary adult human epidermal keratinocyte (HEKa) cells in the presence of human keratinocyte growth supplement (HKGS) increased pERK, representing activation of the MAPK pathway. The increase in pERK was similar with the addition of the BRAF inhibitor vemurafenib, which is known to induce paradoxical MAPK activation, and it was higher with LUT014, which had been selected to maximize the paradoxical increase in pERK **(****Fig. 1D****).** Addition of the EGFR kinase inhibitor erlotinib, or the antibody blocking EGFR cetuximab, partially inhibited the HKGS stimulation of the MAPK pathway in HEKa cells, demonstrated by decreased pERK. The addition of LUT014 abrogated the inhibitory effect of both EGFR inhibitors, increasing pERK to levels comparable to that seen with HKGS alone **(****Fig. 1E****).** As it has been reported that at higher concentrations of a BRAF inhibitor the paradoxical activation effects decrease, the effects of LUT014 on the proliferation of the *KRAS* G12C mutated human pancreatic cancer cell line MIA-PaCa-2 were tested (Deer, E.L., et al. Pancreas 39, 425-435, 2010). Increasing concentrations LUT014 induced a concentration-dependent increased proliferation of MIA-PaCa-2 cells, with a peak proliferation at 0.041 µM. Surprisingly, at higher concentrations, LUT014 induced a concentration-dependent decreased proliferation inducing MIA-PaCa-2 growth arrest at the highest concentrations **(****Fig. 1F**). Therefore, LUT014 tested *in vitro* reverses the MAPK pathway inhibition induced by EGFR inhibitors, through a bell-shaped paradoxical MAPK activation and downstream cell proliferation.

To test if topical administration of LUT014 would benefit patients with acneiform rash induced by anti-EGFR therapies, 10 patients with metastatic colorectal carcinoma who developed grade 1 or 2 skin rash while on therapy with cetuximab or panitumumab were enrolled (**Table 4).**

**Table 4. Patient characteristics**

| | | **0.3 mg/g LUT014 Gel (N = 3)** | **1.0 mg/g LUT014 Gel (N = 4)** | **2.5 mg/g LUT014 Gel (N = 3)** | **Total (N = 10)** |
|---|---|---|---|---|---|
| **Age (years)** | | | | | |
| | Median | 67 | 50 | 55 | 54 |
| | Range | 53 - 70 | 42 - 66 | 49 - 67 | 42 - 70 |

| **Gender** | | | | | |
|---|---|---|---|---|---|
| | Female | 2 | 0 | 0 | 2 |
| | Male | 1 | 4 | 3 | 8 |

| **Race** | | | | | |
|---|---|---|---|---|---|
| | Black or African American | 0 | 1 | 0 | 1 |
| | White | 3 | 3 | 2 | 8 |
| | Other | 0 | 0 | 1 | 1 |

| **Stage** | | | | | |
|---|---|---|---|---|---|
| | IVa | 2 | 3 | 1 | 6 |
| | IVb | 1 | 1 | 2 | 4 |

| **EGFR inhibitor Antibody** | | | | | |
|---|---|---|---|---|---|
| | cetuximab | 1 | 2 | 1 | 4 |
| | panitumumab | 2 | 2 | 2 | 6 |

| **EGFR inhibitor Treatment of metastatic colorectal carcinoma - Number of Days Prior to Screening Visit that Treatment Initiated** | | | | | |
|---|---|---|---|---|---|
| | Median | 57 | 106 | 42 | 57 |
| | Range | 3-79 | 28 - 265 | 8 - 69 | 3 to 265 |

| | | | | | |
|---|---|---|---|---|---|
| a. The data summarized in this table are based on draft data collected; the data have not undergone quality control review and are subject to change. | | | | | |

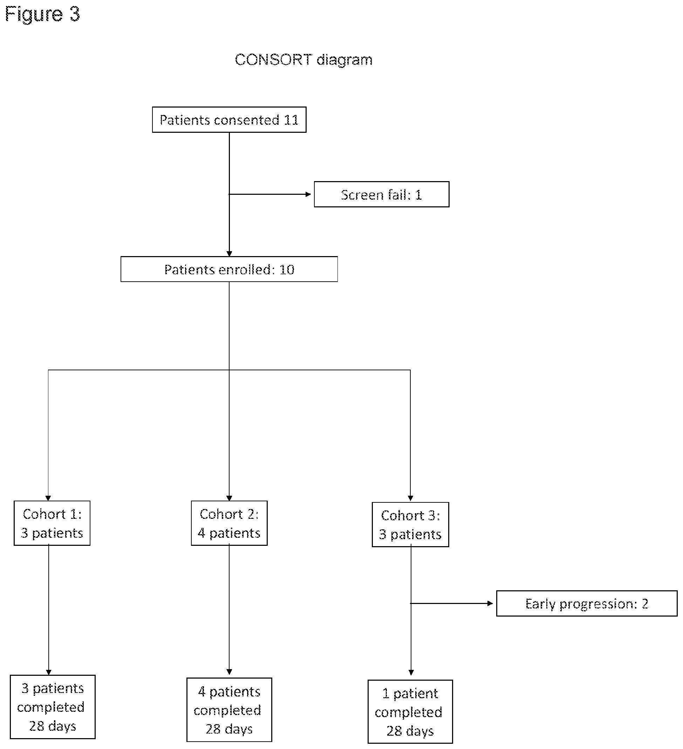

Three patients were assigned to the 0.3 mg/g dose cohort 1, four to the 1.0 mg/g dose cohort 2, and three to the 2.5 mg/g dose cohort 3 **(****Fig. 3****).** Table 5 provides the baseline assessment of EGFR inhibitor-induced skin toxicity and Table 6 the treatment adherence.

**Table 5: Baseline characteristics of the skin rash.**

| | | **0.3 mg/g LUT014 Gel (N = 3)** | **1.0 mg/g LUT014 Gel 2.0 (N = 4)** | **2.5 mg/g LUT014 Gel (N = 3)** | **Total (N = 10)** |
|---|---|---|---|---|---|
| **Grading of Acneiform Lesions by CTCAE Version 5.0 at Baseline (Day 0) - Investigator** | | | | | |
| | Grade 1 | 0 | 2 | 1 | 3 |
| | Grade 2 | 3 | 2 | 2 | 7 |

| **Grading of Acneiform Lesions by MESTT at Baseline (Day 0) - Central Reader (based on photos)** | | | | | |
|---|---|---|---|---|---|
| | Grade 1 | 1 | 0 | 1 | 2 |
| | Grade 2 | 1 | 2 | 1 | 4 |
| | Grade 3 | 1 | 2 | 1 | 4 |

| **FACT-EGFR inhibitor-13 HRQoL Questionnaire - Total Score for First 13 Questions (Skin-Specific)** | | | | | |
|---|---|---|---|---|---|
| | Mean (SD) | 34.7 (4.93) | 40.0 (14.02) | 40.7 (8.33) | 38.6 (9.69) |
| | Median | 37 | 43 | 38 | 38 |
| | Range | 29 - 38 | 23 - 52 | 34 - 50 | 23 - 52 |

| **ECOG Performance Status** | | | | | |
|---|---|---|---|---|---|
| Grade 0 | | 2 | 4 | 1 | 7 |
| Grade 1 | | 1 | 0 | 2 | 3 |

**Table 6. Extent of Exposure**

| | | **0.3 mg/g LUT014 Gel (N = 3)** | **1.0 mg/g LUT014 Gel (N = 4)** | **2.5 mg/g LUT014 Gel (N = 3)** | **Total (N = 10)** |
|---|---|---|---|---|---|
| **Number of Patients that Received at Least 1 Dose** | | 3 | 4 | 3 | 10 |
| **Total Number of Doses Taken** | | | | | |
| | Median | 28 | 28 | 23 | 28 |
| | Range | 27 - 28 | 27 - 29 | 22 - 28 | 22 - 29 |

| **Percentage of Planned Dose Taken (out of 28 )** | | | | | |
|---|---|---|---|---|---|
| | Median | 100.0% | 100.0% | 82.1% | 100.0% |
| | Range | 96.4% - 100.0% | 96.4% - 101.0% | 78.6% - 100.0% | 78.6% - 101.0% |
| | <75% | 0 | 0 | 0 | 0 |
| | 75%≤x<100% | 1 | 1 | 2 | 4 |
| | 100% | 2 | 2 | 1 | 5 |
| | >100% | 0 | 1 | 0 | 1 |

| **Total Treatment Duration (number of days from first to last dose)** | | | | | |
|---|---|---|---|---|---|
| | Median | 28 | 28 | 23 | 28 |
| | Range | 28 - 28 | 28 - 29 | 22 - 28 | 22 - 29 |

| | | | | | |
|---|---|---|---|---|---|
| a. The data summarized in this table are based on draft data collected; the data have not undergone quality control review and are subject to change. | | | | | |

In general, the study drug was well tolerated and no dose limiting toxicity (DLT) or maximum tolerated dose (MTD) was defined. A total of 38 adverse events of any attribution were reported, with comparable numbers for each dose cohort **(Table 7).**

**Table 7. Treatment-related adverse events**

| **Toxicity** | **0.3 mg/g (N = 3)** | | **1.0 mg/g (N = 4)** | | **2.5 mg/g (N = 3)** | | **Total (N = 10)** | |
|---|---|---|---|---|---|---|---|---|
| | All Grades | Grade 3 or 4 | All Grades | Grade 3 or 4 | All Grades | Grade 3 or 4 | All Grades | Grade 3 or 4 |
| Local Pain | 0 | 0 | 0 | 0 | 1 | 0 | 1 | 0 |
| Dry skin | 0 | 0 | 0 | 0 | 1 | 0 | 1 | 0 |
| Pruritus | 3 | 0 | 2 | 0 | 1 | 0 | 6 | 0 |

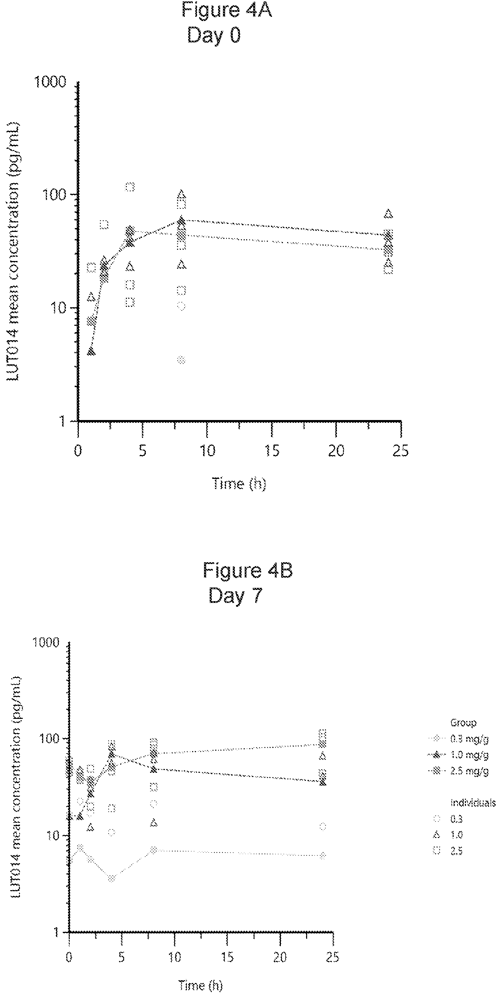

Seven patients experienced an adverse event considered to be related to the study drug, including pruritus, dry skin and stinging sensation (all were grade 1-2 in severity). There were no local or systemic toxicities that have been associated with the clinical use of BRAF inhibitors administered as oral systemic therapies, such as photosensitivity, maculo-papular rash or hyperkeratosis (Belum, et al., Current Oncology Reports 15, 249-259, 2013). Pharmacokinetic analysis using blood samples of patients treated with topical LUT014 showed negligible absorption and minimal systemic exposure, with plasma concentrations in the pg/mL range **(****Figs. 4A-4B****).** These are concentrations that are 3 to 4 orders of magnitude lower than the effective systemic concentrations of other BRAF inhibitors, which are measured in µg/mL (Bollag, et al., Nature 467, 596-599, 2010; Flaherty, et al., N Engl J Med 363, 809-819, 2010; Ribas, et al. Journal of clinical pharmacology 54, 368-374, 2014).

The surprising pharmaco-kinetic results of this disclosure show negligible to minimal systemic absorption of compound of formula IV. This is proof that the topical treatment disclosed here will not cause systemic side-effects and thus avoid the potential risk of the interference with the cancer treatment, when administered systemically.

Three scales were used to evaluate the severity of acneiform lesions during the clinical trial period. The primary readout was based on the National Cancer Institute (NCI) Common Terminology Criteria for Adverse Events (CTCAE) version 5.0 for grading acneiform rash. The clinical trial eligibility criteria were based on having an acneiform skin rash of grade 1 or 2 while on anti-EGFR therapy, and therefore the grading change from baseline with the topical application of LUT014 was measured. The Multinational Association of Supportive Care in Cancer (MASCC) Study Group EGFR Inhibitor-dermatologic adverse event grading scale is a central reviewer-assessment based on the review of pictures provided to one remote reviewer (Wong, et al., Support Care Cancer 21, 2933-2948, 2013). The Functional Assessment of Cancer Therapy (FACT) is a health-related quality of life questionnaire to assess symptoms associated with EGFR inhibitors (Wagner, et al., Support Care Cancer 21, 1033-1041, 2013). The score based on the 13 skin-specific questions (FACT-EGFRI-13) was reported, and was calculated by the change in total score relative to baseline, with a decrease in value representing worsening and an increase value representing improvement of skin-related symptoms.

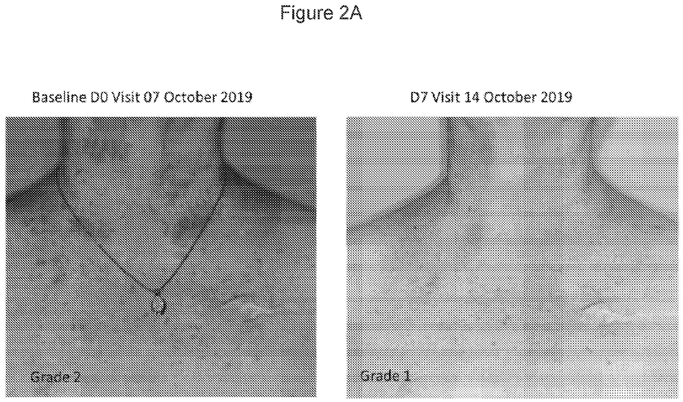

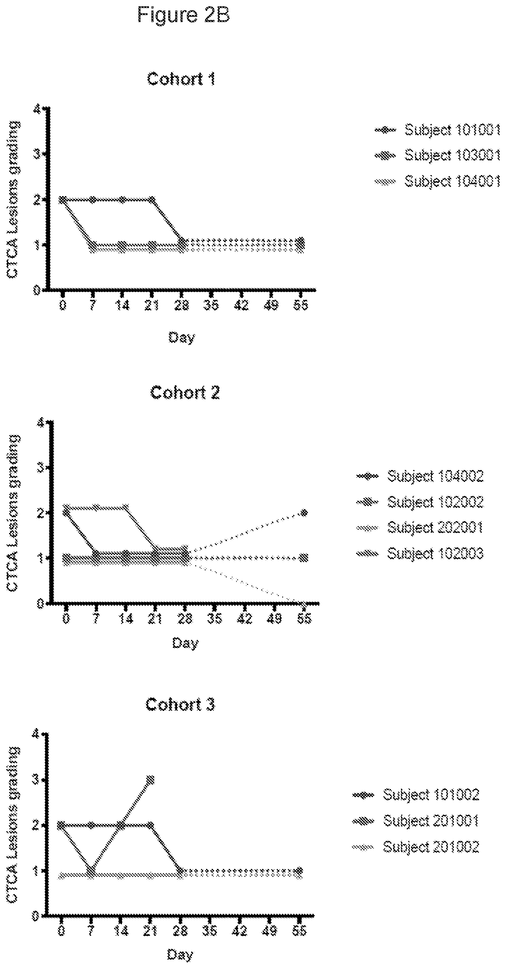

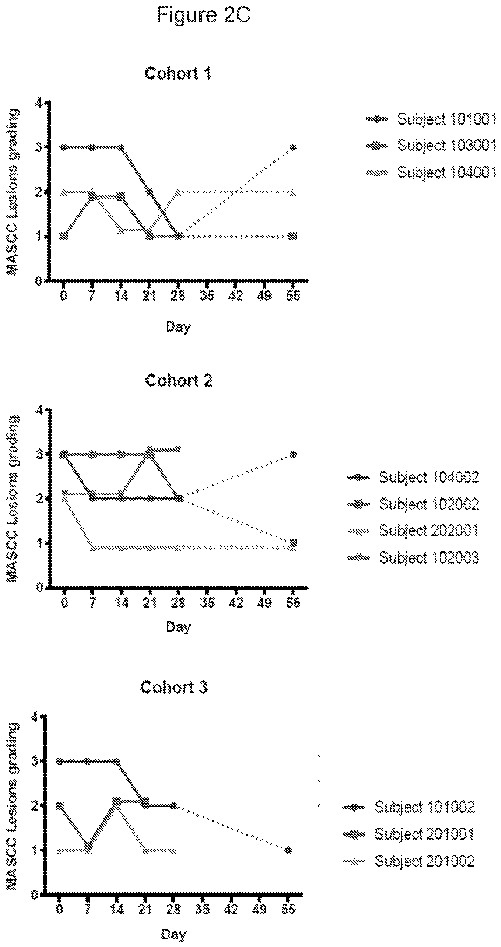

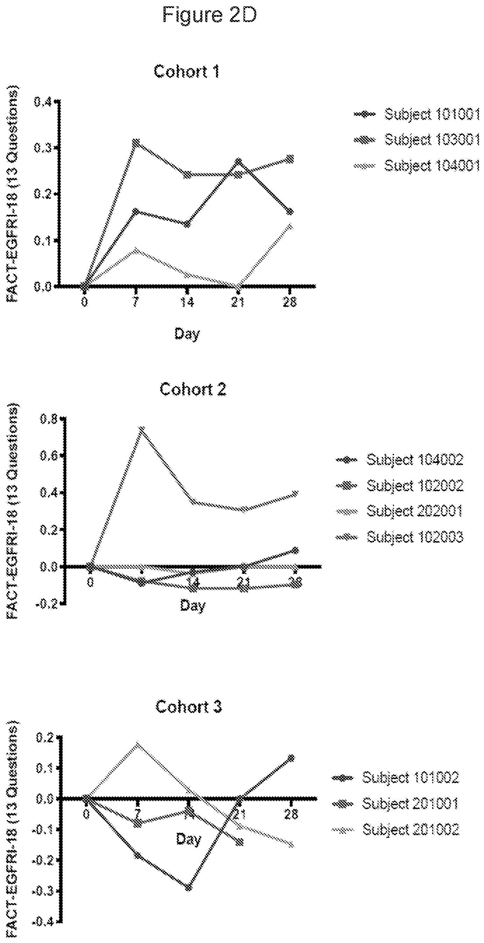

The three patients in cohort 1 entered the study with grade 2 acneiform rash, and all improved to grade 1 during the 28-day treatment period, with two of the patients showing improvement within the first week. All three patients continued to have a sustained benefit by day 55, a month after administration had terminated **(****Figs. 2A-2B****).** Using the central reviewer MASCC grading, two patients improved and one patient did not change in the central assessment during the treatment period **(****Fig. 2C****).** With the FACT-EGFRI-13 skin-related symptom questionnaire, the three patients had symptomatic improvement during the 28-day treatment period **(****Fig. 2D****).** Cohort 2 accrued four patients, two with grade 2 at baseline that improved to grade 1 during the 28-day treatment period (one improved at one week and the other at the second week), and two with grade 1 at baseline that did not change in grading during the treatment period **(****Fig. 2B****).** Using the MASCC grading, three patients improved grading during the treatment period while one worsened **(****Fig. 2C****).** Using the FACT-EGFRI-13 symptom questionnaire, two patients with baseline grade 2 rash improved symptoms, and two patients with baseline grade 1 rash reported stable skin-related symptoms **(****Fig. 2D****).** There were three patients in cohort 3, two starting with grade 2 and one with grade 1 toxicity. Of them, one improved, another was stable and one patient had worsening acneiform skin rash during the 28-day treatment period, leading to discontinuation of therapy after study day 21 **(****Fig. 2B****).** The same course was evident when using the MASCC grading **(****Fig. 2C****).** Using the FACT-EGFRI-13 symptom questionnaire, one patient improved and two worsened **(****Fig. 2D****).**

Therefore, acneiform skin rash induced by anti-EGFR therapy improved in all patients in the lowest dose cohort of topical LUT014 when analyzed using three assessment criteria, which included both objective and patient-reported outcomes. In cohort 2 there was also evidence of acneiform rash improvement, whereas in the highest dose cohort there was no consistent evidence of improvement. This clinical data is reminiscent of the bell-shaped curve of paradoxical MAPK activation with BRAF inhibitors, with increase in pERK at certain concentrations of the BRAF inhibitor and decreased pERK at higher concentrations (Solit, et al, 2014; Holderfield, 2014). Accordingly, improving a topmost adverse event of EGFR inhibitor therapy with topical LUT014 could allow maintaining quality of life and dose intensity, thereby maximizing the antitumor effects while locally inhibiting dose-limiting skin toxicities.

### Methods

### LUT014 drug characterization

LUT014 has the chemical name of 5-N-(3-(9H-purin-6-yl)pyridin-2-yl)-6-methyl-1-N-(3-trifluoromethoxy) phenyl)isoquinoline-1,5diamine, with a molecular weight (MW) of 528.49 Daltons (Da) and a molecular formula of C₂₇H₁₉F₃N₈O.

Three strengths of LUT014 formulated as an aqueous gel for topical administration were used in the clinical trial: 0.3 mg/g [0.03% weight/weight (w/w)], 1.0 mg/g (0.10% w/w), and 2.5 mg/g (0.25% w/w).

### Preclinical in vitro testing of pERK induction

Primary HEKa, isolated from adult skin of a single donor and cryopreserved at the end of primary culture (Gibco catalog number C0055C, Thermo Fisher Scientific, Waltham, MA), were cultured in T25 flasks at 37°C, 5% CO₂ in growth medium (Epilife^{™} CF Kit, Gibco) supplemented with 60 µM calcium (Gibco) and HKGS (Gibco). After at least three passages, cells were harvested with trypsin when flask reached 70-80% confluence, and seeded in 10 cm culture dishes (250,000 cells/dish) in 9 ml of growth medium overnight. To analyze the effect of HKGS or a BRAF inhibitor on pERK, cells were starved for 2.5 hours in 5 mL of starvation medium without HKGS, and then individual plates were incubated for two hours with DMSO 0.1%, HKGS, vemurafenib 0.3 µM (0.146 µg/mL), or LUT014 0.3 µM (0.158 µg/mL). To analyze the effects of LUT014 to re-activate pERK in HKGS-activated HEKa cells cultured in an EGFR inhibitor, cells were seeded in 60 mm dishes (120,000 cells/dish) in 4 ml growth medium. After overnight incubation, cells were starved for 2.5 hours in 3 mL of starvation medium, and then individual plates were incubated for two hours with DMSO 0.1%, HKGS, HKGS plus erlotinib stock solution (10 µM) plus DMSO (0.1%), HKGS plus erlotinib stock solution (10 µM) plus LUT014 (0.3 µM; 0.158 µg/mL), HKGS plus cetuximab stock solution (50 µg/mL) plus DMSO (0.1%), or HKGS plus cetuximab stock solution (50 µg/mL) plus LUT014 (0.3 µM; 0.158 µg/mL). Cells were harvested with 1 ml cold PBS containing EDTA-free, phosphatase inhibitor cocktail (MilliporeSigma, Burlington, MA) using a cell scraper. The cell suspensions were then used for Western Blot as previously described (Reuveni, et al. Cancer Res 73, 4383-4394, 2013). The monoclonal anti-diphosphorylated ERK-1&2 (clone MAPK-YT Mouse Ascites Fluid, MilliporeSigma), and the anti-ERK 2 antibody (C-14, rabbit polyclonal IgG, Santa Cruz Biotechnology, Dallas, TX) were used. The membranes were developed with WesternBright Sirius chemiluminescent substrate (Advansta, San Jose, CA) and photographed with an Omega LumG imager (Aplegen, Gel Company, San Francisco, CA). The signals of phosphorylated ERK1/2 and total ERK2 were quantified using ImageJ software and the phosphorylated ERK1/2/total ERK2 ratio was calculated. For the quantification, equal sized rectangles were drawn around the bands of the phosphorylated ERK1/2 and the total ERK2 and the pixels in each rectangle were measured.

### Preclinical in vitro testing of cellular proliferation induction

MIA-PaCa-2 cells (ATCC catalog number CRM-CRL-1420, Manassas, VA) were cultured at 37°C in T75 flasks in growth medium DMEM (ATCC) supplemented with 10% FBS and 2.5% horse serum. After 4 days, when the flasks reached 50% confluence, cells were harvested with trypsin and seeded in 96 well plates at a concentration of 5000 cells/well, in a total volume of 0.1 ml/well starvation medium and incubated 24 hours at 37°C, 5% CO₂. The next morning, medium was replaced with 180 µl growth medium (containing 10% FBS and 2.5% horse serum) and 20 µl of each concentration of LUT014, DMSO or PBS. Each concentration of each compound was added to triplicate wells. The plates were incubated for 72 hours at 37°C, 5% CO₂. Cellular proliferation was measured using the ATP-lite proliferation assay (Perkin Elmer, Waltham, MA) following the manufacturer's instructions, with the luminescence measured using a CLARIOstar reader (BMG Labtech, Ortenberg, Germany), and analyzed with the ATPlite TOP program (Perkin Elmer).

### Clinical study design

This was an open-label, dose escalation study of LUT014 gel topically administered for four weeks. A 2-week screening period (day -14 to day -1) was followed by a 4-week treatment period (day 0 to day 27). Patients applied the study drug once daily to their face, neck, chest, and upper back. The post-treatment safety follow-up period ran from day 28 to day 55. Additionally, two long term safety follow up visits were done 3 and 6 months after completion of treatment, including a dermatological exam to detect any potential BRAF inhibitor skin toxicities within the treatment area.

Three dose levels of LUT014 gel were tested: 0.3 mg/g, 1.0 mg/g and 2.5 mg/g. The dose of LUT014 was escalated in sequential dose cohorts of 3 to 6 patients, in a conventional 3+3 escalating dose design. Escalation to the next dose level was permitted if no DLT was reported by the three patients in the cohort within 4 weeks of receiving the first dose of LUT014. If one out of three patients developed a dose limiting toxicity (DLT), additional three patients were planned to be treated with study drug at the same dose level. At any given dose level, if more than one out of the three or six patients experienced a DLT within 4 weeks of receiving the first dose of LUT014, the dose level was determined to have exceeded the MTD, and the MTD was set to be the next lower dose.

A DLT was defined as any treatment-emergent grade 2 or higher clinically significant adverse event, with the exception of a transient grade 2 adverse event (i.e., one that returns to grade 1 or baseline within 7 days) or grade 2 adverse event that was manageable with standard of care (i.e., one that returns to grade 1 or baseline within 14 days), that occurred within 4 weeks of the first dose of study drug for which there was a reasonable possibility that LUT014 had caused the event. Upon completion of the 4-week treatment period of the third patient in each dosing cohort, a Safety Review Committee (SRC) meeting was set to review the safety data and to provide recommendation/decision on dose escalation/de-escalation, or expansion of dosing cohort.

### Study patients

Eligible patients were 18 years or older, with a diagnosis of a metastatic colorectal carcinoma treated with cetuximab (Erbitux^{®}) or panitumumab (Vectibix^{®}) and who had developed a grade 1 or 2 acneiform skin toxicity, based on CTCAE version 5.0-skin and subcutaneous tissue disorders grading scale. The anti-EGFR treatment must had been initiated within 12 weeks prior to the screening visit and continued through the day 55 visit at the same dose, except if required per the dose modification section of the approved product labeling. Unless required to treat an adverse event, patients were not allowed to receive any systemic antibiotic treatment within 7 days prior to screening until 1 month after the completion of the treatment with the study drug (day 55). In addition, topical corticosteroids to the targeted treatment areas, as well as systemic corticosteroids therapy, except for low dose systemic corticosteroids given as part of standard of care for the prevention or treatment of chemotherapy-induced nausea and vomiting, were prohibited 14 days prior to study entry throughout day 55 in the study. Eleven patients were screened, and one did not proceed due to not meeting the eligibility criteria. Two patients in cohort 2 had started anti-EGFR therapy greater than 12 weeks prior to the screening visit, and one patient in cohort 3 who was treated with systemic antibiotic within 7 days prior to screening but 9 days prior to baseline; these three patients were given waivers by the study sponsor and the local IRBs to participate in the trial, and thus all 10 patients were enrolled.

### Study objectives

The primary objective of the study was to evaluate the safety and tolerability of LUT014 topically applied daily for 4 weeks in patients with metastatic colorectal carcinoma with anti-EGFR therapy-induced acneiform lesions. The secondary objectives were to assess the pharmacokinetics of the LUT014 in plasma after a single administration and after 8 days of daily administrations and to evaluate the preliminary efficacy of 4 weeks treatment of the LUT014 on anti-EGFR therapy-induced acneiform lesions. Patients underwent oncologic treatment efficacy assessments of their colorectal cancer as per the institutional standard of care.

### Safety and tolerability assessments

Safety and tolerability were assessed primarily based on the adverse events from day 0 to day 55. In addition to the adverse events assessment, the safety measurements included physical examination, vital signs and body weight, 12-lead ECG, ECOG performance status, safety labs including complete blood count, comprehensive metabolic panel and urinalysis. At 3 and 6 months from study start a dermatologist performed a skin assessment of the areas of the skin where LUT014 gel was applied to screen for potential cutaneous toxicities known to be associated with approved BRAF inhibitors, including erythema nodosum-type rash, photosensitivity, squamous papillomas or warts, keratoacanthomas, cutaneous squamous cell carcinoma, dry skin, and folliculitis or cysts (Welsh, et al., Therapeutic advances in medical oncology 7, 122-136, 2015).

### Pharmacokinetics (PK) analyses

Blood samples for PK analysis were taken from the initial three patients enrolled to each dosing cohort (total 9 patients). PK samples were collected pre-dose on day 0 and day 7 and at 1, 2, 4, 8 and 24 hours post-dose. A qualified liquid chromatography tandem mass spectroscopy LC-MS/MS analytical assay with an LLOQ of 10 pg/ml was used to determine concentrations of LUT014 in plasma. Plasma concentrations below the limit of quantitation (<10 pg/mL) were treated as zero for the calculations of mean (SD).

### Efficacy assessment

Evaluation of the preliminary efficacy of LUT014 gel for the treatment of anti-EGFR therapy-induced acneiform lesions was based on the following: A) Acneiform lesions grading performed locally by the Investigator using the NCI CTCAE version 5.0 skin and subcutaneous tissue disorders grading scale. B) Acneiform lesions grading performed by a central reader, using the MASCC Study Group EGFR Inhibitor-dermatologic adverse event grading scale (Wong, et al., 2013). Grading was based on masked photographs of patients' face, neck, and upper portion of the anterior and posterior chest. C) Patient self-reported quality of life FACT-EGFRI-13 questionnaire (Wagner et al, 2013), assessing the physical and psychological effects of the dermatological symptoms associated with anti-EGFR treatment on study patients. Only the first 13 questions of the FACT-EGFRI that are relevant for the skin were evaluated. Data from all patients who received at least one dose of study drug were included in the safety and efficacy analyses.

### Standard protocol approvals, registration, and patient consents

Informed consent was obtained from all study patients prior to any study related procedure. The study was conducted in accordance with the applicable regulatory and International Council for Harmonization-Good Clinical Practice requirements, the ethical principles that have their origin in the principles of the Declaration of Helsinki, and the local laws and regulations of the study sites regarding the conduct of clinical trials and the protection of human patients.

### Statistical analyses

Tabular summaries of the secondary safety assessments included descriptive statistics (i.e., mean, standard deviation, median, and range for continuous data and frequency for categorical data) for each visit assessed, as well as for the calculated changes from baseline. For the pharmacokinetics analyses, plasma LUT014 concentration-time profiles were analyzed by non-compartmental methods and PK parameters were calculated by standard equations, when plasma levels are achieved. PK analyses produced using Phoenix^{®} WinNonLin (Certara, Princeton, NJ). The efficacy assessments NCI CTCAE skin and subcutaneous tissue disorders grading scale, MASCC Study Group EGFR Inhibitor-dermatologic adverse event grading scale, FACT-EGFRI-13 questionnaire, were summarized by treatment group using descriptive statistics as appropriate (i.e., mean, standard deviation, median, and range for continuous data and frequency for categorical data). All analyses were performed using SAS^{®} Software (version 9.2, SAS Institute, Cary, NC).

## Claims

1. A topical pharmaceutical composition for use in a method of treating and/or preventing EGFR inhibitor, PI3K inhibitor, MEK inhibitor and/or HER dimerization inhibitor - induced acneiform lesions in a subject in need thereof, the method comprising:
administering the topical pharmaceutical composition once a day for 4 to 6 weeks to the skin of the subject in need thereof, the topical pharmaceutical composition comprising from 0.03 % weight/weight to 0.1 % weight/weight of a compound of Formula IV 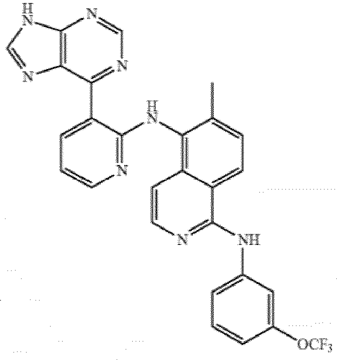
and a pharmaceutically acceptable carrier or excipient.

2. The composition for use of claim 1, wherein the acneiform lesions are EGFR inhibitor-induced acneiform lesions.

3. The composition for use of claim 1, wherein the step of administering comprises applying the topical pharmaceutical composition for four to six weeks to the skin of the subject in need thereof.

4. The composition for use of claim 1, wherein the topical pharmaceutical composition is in the form of a gel, a hydrogel, an ointment, a cream, a spray, a dermal patch, a foam, a lotion or a liquid.

5. The composition for use of claim 1, wherein the subject in need thereof is a subject has a cancer treated with EGFR inhibitor, PI3K inhibitor, MEK inhibitor and/or HER dimerization inhibitor.

6. The composition for use of claim 1, wherein the subject in need thereof has grade 1 acneiform lesions.

7. The composition for use of claim 1, wherein the subject in need thereof has grade 2 acneiform lesions.

8. The composition for use of claim 1, wherein the subject in need thereof has grade 3 acneiform lesions.

9. The composition for use of claim 1, wherein the subject in need thereof has grade 4 acneiform lesions.

10. The composition for use of claim 1, wherein the composition reduces the severity or prevents escalation of the acneiform lesions.

11. The composition for use of claim 1, wherein the composition reduces the severity of the acneiform lesions from grade 4 to grade 3, 2, 1, or 0.

12. The composition for use of claim 1, wherein the composition reduces the severity of the acneiform lesions from grade 3 to grade 2, 1, or 0.

13. The composition for use of claim 1, wherein the composition reduces the severity of the acneiform lesions from grade 2 to grade 1, or 0.

14. The composition for use of claim 1, wherein the composition reduces the severity of the acneiform lesions from grade 1 to grade 0.

15. The composition for use of claim 1, wherein the acneiform lesions are induced by treatment with gefitinib, erlotinib, lapatinib, cetuximab, panitumumab, vandetanib, necitumumab, osimertinib, afatinib, neratinib, pertuzumab or combinations thereof.

16. The composition for use of claim 1 wherein the topical pharmaceutical composition comprises 0.03 % weight/weight of the compound of Formula IV.

## Patentansprüche

1. Topische pharmazeutische Zusammensetzung zur Verwendung in einem Verfahren zur Behandlung und/oder Prävention von EGFR-Inhibitor-, PI3K-Inhibitor- , MEK-Inhibitor- und/oder HER-Dimerisierungsinhibitor-induzierten akneartigen Läsionen bei einem Patienten, der diese benötigt, wobei das Verfahren umfasst:
Auftragen der topischen pharmazeutischen Zusammensetzung einmal täglich für 4 bis 6 Wochen auf die Haut des Patienten, der diese benötigt, wobei die topische pharmazeutische Zusammensetzung 0,03 Gew.-% bis 0,1 Gew.-% einer Verbindung der Formel IV 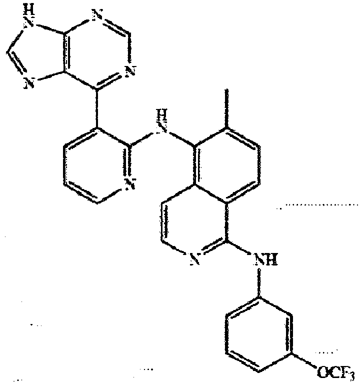
und einen pharmazeutisch annehmbaren Träger oder Hilfsstoff umfasst.

2. Zusammensetzung zur Verwendung nach Anspruch 1, wobei die akneartigen Läsionen EGFR-Inhibitor-induzierte akneartige Läsionen sind.

3. Zusammensetzung zur Verwendung nach Anspruch 1, wobei der Schritt zum Verabreichen das Auftragen der topischen pharmazeutischen Zusammensetzung für vier bis sechs Wochen auf die Haut des Patienten, der diese benötigt, umfasst.

4. Zusammensetzung zur Verwendung nach Anspruch 1, wobei die topische pharmazeutische Zusammensetzung in Form eines Gels, eines Hydrogels, einer Salbe, einer Creme, eines Sprays, eines Hautpflasters, eines Schaums, einer Lotion oder einer Flüssigkeit vorliegt.

5. Zusammensetzung zur Verwendung nach Anspruch 1, wobei der Patient, der diese benötigt, ein Patient ist, der Krebs hat, der mit einem EGFR-Inhibitor, einem PI3K-Inhibitor, einem MEK-Inhibitor und/oder einem HER-Dimerisierungsinhibitor behandelt wird.

6. Zusammensetzung zur Verwendung nach Anspruch 1, wobei der Patient, der diese benötigt, akneartige Läsionen des Grades 1 aufweist.

7. Zusammensetzung zur Verwendung nach Anspruch 1, wobei der Patient, der diese benötigt, akneartige Läsionen des Grades 2 aufweist.

8. Zusammensetzung zur Verwendung nach Anspruch 1, wobei der Patient, der diese benötigt, akneartige Läsionen des Grades 3 aufweist.

9. Zusammensetzung zur Verwendung nach Anspruch 1, wobei der Patient, der diese benötigt, akneartige Läsionen des Grades 4 aufweist.

10. Zusammensetzung zur Verwendung nach Anspruch 1, wobei die Zusammensetzung den Schweregrad der akneartigen Läsionen reduziert oder deren Eskalation verhindert.

11. Zusammensetzung zur Verwendung nach Anspruch 1, wobei die Zusammensetzung den Schweregrad der akneartigen Läsionen von Grad 4 auf Grad 3, 2, 1 oder 0 reduziert.

12. Zusammensetzung zur Verwendung nach Anspruch 1, wobei die Zusammensetzung den Schweregrad der akneartigen Läsionen von Grad 3 auf Grad 2, 1 oder 0 reduziert.

13. Zusammensetzung zur Verwendung nach Anspruch 1, wobei die Zusammensetzung den Schweregrad der akneartigen Läsionen von Grad 2 auf Grad 1 oder 0 reduziert.

14. Zusammensetzung zur Verwendung nach Anspruch 1, wobei die Zusammensetzung den Schweregrad der akneartigen Läsionen von Grad 1 auf Grad 0 reduziert.

15. Zusammensetzung zur Verwendung nach Anspruch 1, wobei die akneartigen Läsionen durch Behandlung mit Gefitinib, Erlotinib, Lapatinib, Cetuximab, Panitumumab, Vandetanib, Necitumumab, Osimertinib, Afatinib, Neratinib, Pertuzumab oder Kombinationen davon induziert werden.

16. Zusammensetzung zur Verwendung nach Anspruch 1, wobei die topische pharmazeutische Zusammensetzung 0,03 Gew.-% der Verbindung der Formel IV umfasst.

## Revendications

1. Composition pharmaceutique topique destinée à être utilisée dans un procédé de traitement et/ou de prévention des lésions acnéiformes induites par l'inhibiteur de l'EGFR, l'inhibiteur de PI3K, l'inhibiteur de MEK et/ou l'inhibiteur de la dimérisation de HER chez un sujet en ayant besoin, le procédé comprenant :
l'administration de la composition pharmaceutique topique une fois par jour pendant 4 à 6 semaines sur la peau du sujet en ayant besoin, la composition pharmaceutique topique comprenant de 0,03 % poids/poids à 0,1 % poids/poids d'un composé de 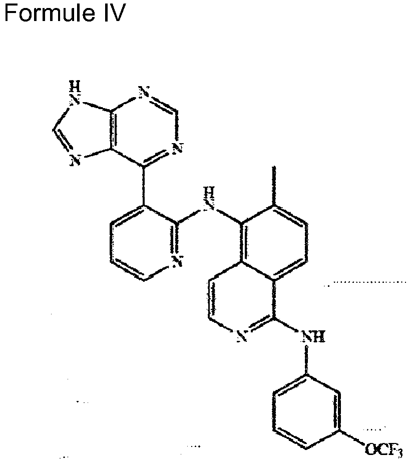
et un support ou excipient pharmaceutiquement acceptable.

2. Composition destinée à être utilisée selon la revendication 1, dans laquelle les lésions acnéiformes sont des lésions acnéiformes induites par un inhibiteur de l'EGFR.

3. Composition destinée à être utilisée selon la revendication 1, dans laquelle l'étape d'administration comprend l'application de la composition pharmaceutique topique pendant quatre à six semaines sur la peau du sujet qui en a besoin.

4. Composition destinée à être utilisée selon la revendication 1, dans laquelle la composition pharmaceutique topique se présente sous la forme d'un gel, d'un hydrogel, d'une pommade, d'une crème, d'un spray, d'un patch dermique, d'une mousse, d'une lotion ou d'un liquide.

5. Composition destinée à être utilisée selon la revendication 1,dans laquelle le sujet en ayant besoin est un sujet qui a un cancer traité avec un inhibiteur de l'EGFR, un inhibiteur de PI3K, un inhibiteur de MEK et/ou un inhibiteur de la dimérisation de HER.

6. Composition destinée à être utilisée selon la revendication 1, dans laquelle le sujet qui en a besoin présente des lésions acnéiformes de grade 1.

7. Composition destinée à être utilisée selon la revendication 1, dans laquelle le sujet en ayant besoin présente des lésions acnéiformes de grade 2.

8. Composition destinée à être utilisée selon la revendication 1, dans laquelle le sujet en ayant besoin présente des lésions acnéiformes de grade 3.

9. Composition destinée à être utilisée selon la revendication 1, dans laquelle le sujet en ayant besoin présente des lésions acnéiformes de grade 4.

10. Composition destinée à être utilisée selon la revendication 1, dans laquelle la composition réduit la gravité ou empêche l'aggravation des lésions acnéiformes.

11. Composition destinée à être utilisée selon la revendication 1, dans laquelle la composition réduit la gravité des lésions acnéiformes du grade 4 au grade 3, 2, 1 ou 0.

12. Composition destinée à être utilisée selon la revendication 1, dans laquelle la composition réduit la gravité des lésions acnéiformes du grade 3 au grade 2, 1 ou 0.

13. Composition destinée à être utilisée selon la revendication 1, dans laquelle la composition réduit la gravité des lésions acnéiformes du grade 2 au grade 1 ou 0.

14. Composition destinée à être utilisée selon la revendication 1, dans laquelle la composition réduit la gravité des lésions acnéiformes du grade 1 au grade 0.

15. Composition destinée à être utilisée selon la revendication 1, dans laquelle les lésions acnéiformes sont induites par un traitement avec le géfitinib, l'erlotinib, le lapatinib, le cétuximab, le panitumumab, le vandétanib, le nécitumumab, l'osimertinib, l'afatinib, le nératinib, le pertuzumab ou des combinaisons de ceux-ci.

16. Composition destinée à être utilisée selon la revendication 1, dans laquelle la composition pharmaceutique topique comprend 0,03 % en poids/poids du composé de Formule IV.
